# EUROPEAN PATENT SPECIFICATION

(11) **EP 3 849 581 B1**
(45) Date of publication and mention of the grant of the patent: **04.11.2026**
(21) Application number: 19859836.9
(22) Date of filing: 10.09.2019
(51) Int. Cl.: A61K 38/17, A61P 11/00, C07K 7/06, C07K 14/705, A61K 9/14

(54) **DRY POWDER FORMULATION OF CAVEOLIN-1 PEPTIDE FOR USE IN THE TREATMENT OF LUNG DISORDERS**
TROCKENPULVERFORMULIERUNG VON CAVEOLIN-1-PEPTID ZUR VERWENDUNG IN DER BEHANDLUNG VON LUNGENERKRANKUNGEN
FORMULATION EN POUDRE SÈCHE DE PEPTIDE DE CAVÉOLINE-1 POUR L'UTILISATION DANS LE TRAITEMENT DE MALADIES PULMONAIRES

(30) Priority: 10.09.2018 US 201862729010 P
(43) Date of publication of application: 21.07.2021
(60) Divisional application: 25216153.4 / 4 830 702
(73) Proprietor: Board of Regents, The University of Texas System, Austin, TX 78701 (US); Rein Therapeutics, Inc., Austin , TX 78758 (US)
(72) Inventor: WILLIAMS, Robert, O., III, Austin, TX 78746 (US); WATTS, Alan, B., Austin, TX 78704 (US); ZHANG, Yajie, Austin, TX 78703 (US); SAHAKIJPIJARN, Sawittree, Austin, TX 78712 (US); CHRISTENSEN, Dale, Austin, TX 78746 (US); KOLENG, John, J., Austin, TX 78746 (US); SHETTY, Sreerama, Tyler, TX 75707 (US)
(74) Representative: Vossius & Partner Patentanwälte Rechtsanwälte mbB
(86) International application number: PCT/US2019/050349
(87) International publication number: WO 2020/055824

(56) References cited:
- WO-A1-2016/138413
- WO-A2-03/016540
- US-A1- 2007 154 404
- US-A1- 2011 218 152
- US-A1- 2013 224 163
- US-A1- 2016 022 577
- US-A1- 2017 128 520
- HENGSAWAS SURASARANG SORAYA ET AL: "Formulation for a novel inhaled peptide therapeutic for idiopathic pulmonary fibrosis", JOURNAL DRUG DEVELOPMENT AND INDUSTRIAL PHARMACY, vol. 44, no. 2, 10 November 2017 (2017-11-10), US, pages 184 - 198, XP055917238, ISSN: 0363-9045, DOI: 10.1080/03639045.2017.1371736
- ZHANG YAJIE ET AL: "Development of an Excipient-Free Peptide Dry Powder Inhalation for the Treatment of Pulmonary Fibrosis", MOLECULAR PHARMACEUTICS, 8 January 2020 (2020-01-08), US, XP055919000, ISSN: 1543-8384, DOI: 10.1021/acs.molpharmaceut.9b01085
- MATTHIAS SCHMITZ ET AL: "Effect of Cavtratin, a Caveolin-1 Scaffolding Domain Peptide, on Oligodendroglial Signaling Cascades", CELLULAR AND MOLECULAR NEUROBIOLOGY, KLUWER ACADEMIC PUBLISHERS-PLENUM PUBLISHERS, NE, vol. 31, no. 7, 27 April 2011 (2011-04-27), pages 991 - 997, XP019955941, ISSN: 1573-6830, DOI: 10.1007/S10571-011-9694-1
- ANONYMOUS: "Caveolin-1 scaffolding domain peptide - ALX-153-064 - Enzo Life Sciences", 28 August 2018 (2018-08-28), pages 1 - 2, XP055919016, Retrieved from the Internet <URL:http://web.archive.org/web/20180828064451/https://www.enzolifesciences.com/ALX-153-064/caveolin-1-scaffolding-domain-peptide/> [retrieved on 20220509]
- IRNGARTINGER M ET AL: "Pulmonary delivery of therapeutic peptides via dry powder inhalation: effects of micronisation and manufacturing", EUROPEAN JOURNAL OF PHARMACEUTICS AND BIOPHARMACEUTICS, ELSEVIER SCIENCE PUBLISHERS B.V., AMSTERDAM, NL, vol. 58, no. 1, 1 July 2004 (2004-07-01), pages 7 - 14, XP004519822, ISSN: 0939-6411, DOI: 10.1016/J.EJPB.2004.03.016
- K.R. VANDANA ET AL: "An overview on in situ micronization technique – An emerging novel concept in advanced drug delivery", SAUDI PHARMACEUTICAL JOURNAL, vol. 22, no. 4, 1 September 2014 (2014-09-01), AMSTERDAM, NL, pages 283 - 289, XP055293544, ISSN: 1319-0164, DOI: 10.1016/j.jsps.2013.05.004
- DE BOER A. H. ET AL: "Dry powder inhalation: past, present and future", EXPERT OPINION ON DRUG DELIVERY, vol. 14, no. 4, 3 April 2017 (2017-04-03), GB, pages 499 - 512, XP055919217, ISSN: 1742-5247, Retrieved from the Internet <URL:https://tandfonline.com/doi/pdf/10.1080/17425247.2016.1224846> DOI: 10.1080/17425247.2016.1224846
- PHILLIPS E ET AL: "SIZE REDUCTION OF PEPTIDES AND PROTEINS BY JET-MILLING", RESPIRATORY DRUG DELIVERY, XX, XX, 1 January 1998 (1998-01-01), pages 161 - 168, XP000957638
- SHETTY NIVEDITA ET AL: "Physical stability of dry powder inhaler formulations", EXPERT OPINION ON DRUG DELIVERY, vol. 17, no. 1, 2 January 2020 (2020-01-02), GB, pages 77 - 96, XP093111774, ISSN: 1742-5247, DOI: 10.1080/17425247.2020.1702643
- SEBASTIAN P. HERTEL ET AL: "Protein stability in pulmonary drug delivery via nebulization", ADVANCED DRUG DELIVERY REVIEWS, vol. 93, 1 October 2015 (2015-10-01), Amsterdam , NL, pages 79 - 94, XP055726457, ISSN: 0169-409X, DOI: 10.1016/j.addr.2014.10.003

## Description

### BACKGROUND OF THE INVENTION

### 1. Field of the Invention

The present invention relates generally to the fields of molecular biology, pharmaceutics and medicine. Specifically, the present invention concerns a pharmaceutical composition for use in the treatment of an inflammatory disorder or fibrotic condition of the lungs as set out in claim 1.

### 2. Description of Related Art

During lung injury, p53 expression increases, inducing plasminogen activator inhibitor-1 (PAI-1) while inhibiting expression of urokinase-type plasminogen activator (uPA) and its receptor (uPAR), resulting in apoptosis of lung epithelial cells (LECs). The mechanism of injury involves cell surface signaling interactions between uPA, uPAR, caveolin-1 ("Cav-1") and B1-integrin (Shetty *et al.,* 2005). Compositions that modulate these interactions could be used in methods for inhibiting apoptosis of injured, diseased or damaged tissues. For example, for treating inflammation or fibrotic conditions such as pulmonary fibrosis. Thus, there is a need for polypeptides that could be used to prevent or treat lung injuries and diseases and, in particular, stable formulations and simple methods for therapeutic delivery of such polypeptides.

WO 2016/138413 A1 relates to a specific composition comprising a nebulized solution of a biologically active caveolin-1 (Cav-I) scaffolding domain polypeptide.

Surasarang et al. reported in the Journal of Drug Development and Industrial Pharmacy 2018, 44(2), pages 184 to 198, on the formulation for a novel inhaled peptide therapeutic for idiopathic pulmonary fibrosis.

### SUMMARY OF THE INVENTION

The present invention is as defined in the claims and thus relates to a pharmaceutical composition for use in the treatment of an inflammatory disorder or fibrotic condition of the lungs, the pharmaceutical composition for use comprising a dry powder of a peptide, or a pharmaceutically acceptable salt thereof, wherein the peptide is the amino acid sequence of SEQ ID NO: 2, wherein the pharmaceutical composition for use is in the form of a dry powder formulation and to be administered using a dry powder inhaler, and wherein the pharmaceutical composition for use is excipient free.

The dry powder as comprised in the pharmaceutical composition for use may be produced by a milling process. or may be produced by a spray-drying process. Said dry powder may be produced by air jet milling, ball milling, or wet milling. Said dry powder as comprised in the pharmaceutical composition for use may comprise less than 1% (by weight) of water. The pharmaceutical composition for use is excipient free. The pharmaceutical composition for use is formulated for lung delivery. Specifically, the pharmaceutical composition for use is in the form of a dry powder formulation and to be administered using a dry powder inhaler. The pharmaceutical composition for use may be formulated for inhalation pressurized metered dose inhalation.

The pharmaceutical composition for use may comprise a water content of less than 10%, 9%, 8%, 7%, 6% or 5%. The pharmaceutical composition for use comprises a water content of 0.01% to 10%, 0.1% to 10%, 1.0% to 8% or 1% to 5%. The pharmaceutical composition for use may comprise an average particle size of less than 10 µm. The average particle size may be 0.01 µm to 10 µm; 0.1 µm to 8 µm; 0.5 µm to 7 µm or 1 µm to 5 µm.. At least 50%, 55%, 60%, 65% or 70% of the pharmaceutical composition for use may comprise comprises a particle size of 1 µm to 5 µm. The pharmaceutical composition for use of the peptide (*e.g.,* CSP7) may be composed of at least 70% (*e.g.* 70%-80%) particles having a size of 1 µm to 5 µm. In preferred aspects, at least 70%, 75%, 80%, or 85% (*e.g.,* 75% - 95%) of particles in the pharmaceutical composition for use are less than 5 µm in size.

The present invention relates to a pharmaceutical composition for use in the treatment of an inflammatory disorder or fibrotic condition of the lungs, the pharmaceutical composition for use comprising a dry powder of a peptide, or a pharmaceutically acceptable salt thereof, wherein the peptide is the amino acid sequence of SEQ ID NO: 2; wherein the pharmaceutical composition for use is in the form of a dry powder formulation and to be administered using a dry powder inhaler, and the pharmaceutical composition for use is excipient free. The subject to be treated with the pharmaceutical composition described in the claim has an inflammatory disorder or fibrotic condition of the lungs . The subject may have pulmonary inflammation, acute lung injury, lung infection. or pulmonary inflammation. The may have chronic obstructive pulmonary disorder (COPD), an acute lung injury or infection, a lung infection, a chemical-induced lung injury, plastic bronchitis, asthma, acute respiratory distress syndrome (ARDS), inhalational smoke induced acute lung injury (ISALI), bronchiolitis, , bronchiolitis obliterans, a fibrotic condition of the lungs, interstitial lung disease, or idiopathic pulmonary fibrosis (IPF) or lung scarring. The pharmaceutical composition is for use of the present invention may be used in the treatment of idiopathic pulmonary fibrosis (IPF)

Administering at least one additional anti-fibrotic therapeutic is envisaged. For example, the at least one additional anti-fibrotic is NSAID, steroid, DMARD, immunosuppressive, biologic response modulators, or bronchodilator. The subject may be a human.

### BRIEF DESCRIPTION OF THE DRAWINGS

The following drawings form part of the present specification and are included to further demonstrate certain aspects of the present invention. The invention may be better understood by reference to one or more of these drawings in combination with the detailed description of specific embodiments presented herein.
**FIG. 1****: Scanning Electron Microscopy Images of CSP7 Bulk Powder.** Powder samples were sputtered on the sample tray and spread by blowing compressed nitrogen. Samples were viewed using a Hitachi S5500 SEM/STEM scanning electron microscope. Scale bars are pictured at the lower right of images.
**FIG. 2****: Optical Microscopy Images of CSP7 Bulk Powder.** Powder samples were sputtered on the glass slides and viewed using a Leica optical microscope (Leica CTR6500). Scale bars are indicated in the lower left hand corner of each image. Arrows point to agglomerated particles of neat CSP7 powder.
**FIG. 3****: X-ray Powder Diffraction of CSP7 Bulk Powder Particles.** CSP7 bulk powder particles were evaluated using X-ray powder diffraction to determine the crystallinity. Powder is measured from 2 to 40 2θ degrees using a step size of 0.025 2θ degrees and a speed of 2 degree/min.
**FIG. 4****: Polarized Light Microscopy of CSP7 Bulk Powder.** Crystallinity was evaluated by polarized light microscopy. Pictured is a representative image. White arrows point to crystalline regions.
**FIG. 5****: Differential Scanning Calorimetry of CSP7 Bulk Powder.** Bulk CSP7 powder was analyzed by differential scanning calorimetry using TA instrument Q20 Differential Scanning Calorimetry. Pictured is a curve from modulated-DSC with a frequency of 1°C/60s and a rate of 2°C/min, ramping from 25 to 300°C.
**FIG. 6****: Thermogravimetric Analysis of CSP7 Bulk Powder.** Thermogravimetric analysis was performed using a Mettler Thermogravimetric Analyzer, Model TGA/DSC. Pictured is the TGA curve. The heating rate was set to 10°C/min, ramping from 25 to 500°C.
**FIG. 7****: Dynamic Vapor Sorption of CSP7 Bulk Powder.** Bulk CSP7 powder was run on a Surface Measurement Systems DVS instrument for a full sorption/desorption cycle from 0% to 90% relative humidity in steps of 10% at 25 °C. Moisture desorption at 0% humidity and mass change at 90% humidity are indicated.
**FIG. 8****: Particle Size Distribution of Spray Dried CSP7.** CSP7 was mixed with either leucine, trehalose, sodium citrate, or leucine and trehalose, and spray dried. Particle size was evaluated by Malvern Mastersizer 2000 (laser light diffraction, Fraunhofer approximation; dispersive air pressure: 3.0 Bar). Pictured are curves of each spray dried mixture.
**FIG. 9****: Visual observation of homogenized CSP7 suspension.** A. Ethanol processed with the highest power of rotor-stator for 1 min; B. Unprocessed CSP7-ethanol suspension, red arrow indicates large particles/aggregates; C. CSP7-ethanol suspension processed with the highest power of rotor-stator for 1 min, suspension turns dark grey; D. CSP7-ethanol suspension processed with the lowest power of rotor-stator for 1 min, suspension turns light grey.
**FIG. 10****: Optical Microscopy of Air Jet Milled CSP7 Powder.** Powder samples collected from the indicated locations of the air jet mill were imaged by optical microscopy. Pictured are representative images.
**FIG. 11****: Scanning Electron Microscopy of Air Jet Milled CSP7 Powder.** Milled CSP7 powder (batch 171027) was imaged by Scanning Electron Microscopy (SEM) under identical conditions to the bulk CSP7 powder. Pictured is a representative SEM image of the milled CSP7 powder.
**FIG. 12****: Light Microscopy of CSP7 Following Thin Film Freezing.** Powder samples were sputtered on the glass slides and viewed using a Leica optical microscope (Leica CTR6500). Scale bars are indicated in the lower left hand corner of each image.
**FIG. 13****: Scanning Electron Microscopy of Spray Dried CSP7.** Particle morphology of spray dried CSP7 mixtures (A = 100% LTI, B = leucine, C = trehalose, d = sodium citrate, e = leucine and trehalose) was examined using SEM. Representative images are pictured.
**FIG. 14****: X-ray Powder Diffraction of Air Jet Milled CSP7 Powder.** X-ray powder diffraction profiles of milled (batch 171027) and unprocessed bulk CSP7 powder are shown. Diffraction curves indicate a decrease in crystallinity in the milled CSP7.
**FIG. 15****: Physical State of Spray Dried CSP7.** Spray dried CSP7 mixtures were examined by **X-ray diffraction.** Curves indicating the crystallinity or lack thereof of each spray dried mixture of CSP7 are shown.
**FIG. 16****: HPLC Analysis of Spray Dried CSP7.** The purity of spray dried CSP7 mixtures were examined by assaying their chemical potency using HPLC.
**FIG. 17****: HPLC analysis of Air Jet Milled CSP7 Stability.** The stability of both unprocessed bulk CSP7 powder, as well as air jet milled CSP7 (batch 171027) were examined by assaying their chemical potency using HPLC. A sample of each was stored in 3 different conditions (4 °C, 25 °C/60 RH, 40 °C/75 RH) with open/closed vial lid before assaying the chemical potency at 5, 15, and 32 days of storage.
**FIG. 18****: Particle deposition of Aerosolized CSP7.** After aerosolization using a Next Generation Impactor (NGI), all collection surfaces were rinsed with known volumes of 20 mM Tris buffer (pH 10.3). Powders deposited in the throat, pre-separator, and stages 1-MOC were extracted separately and measured. Indicated is the percentage of unprocessed or air jet milled (batch 171013) CSP7 powder (collected from collection vessel) deposited in a specific location.
**FIG. 19****: Aerodynamic Particle Size Distribution of Air Jet Milled CSP7.** After aerosolization using a Next Generation Impactor (NGI), all collection surfaces were rinsed with known volumes of 20 mM Tris buffer (pH 10.3). Powders deposited in the capsule, device, adaptor, throat, pre-separator, and stages 1-MOC were extracted separately and measured. The locations within the mill where either unprocessed or air jet milled (batch 171027) CSP7 powder (collected from all fractions of milled powder) was determined. The percentage of the milled powder present in each location is shown.
**FIG. 20****: Aerosol Performance of Spray Dried CSP7 Mixtures.** After aerosolization by NGI, all collection surfaces were rinsed with known volumes of 20 mM Tris buffer (pH 10.3). Powders deposited in the capsule, device, adaptor, throat, pre-separator, and stages 1 - MOC were extracted separately and measured. Indicated is the percentage of powder deposited in a specific location.
**FIG. 21****: Dynamic Vapor Sorption of Air Jet Milled CSP7 Powder.** Milled CSP7 powder (batch 171027) was run on a Surface Measurement Systems DVS instrument for a full sorption/desorption cycle from 0% to 90% relative humidity in steps of 10% at 25 °C. Moisture desorption at 0% humidity and mass change at 90% humidity are indicated.
**FIG. 22****: Thermal analysis of Air Jet Milled CSP7 Powder.** Differential Scanning Calorimetry (DSC) was performed on milled CSP7 (batch 171027) using a calorimeter as above. Pictured is a curve from DSC with a frequency of 1°C/60s and a rate of 2°C/min, ramping from 25 to 300°C.
**FIG. 23****: Thermal Properties of Spray Dried 100% CSP7.** Excipient free spray dried CSP7 was analyzed by modulated differential scanning calorimetry. Pictured is the mDSC curve.
**FIG. 24****: Thermal Properties of Spray Dried CSP7 with Leucine.** Spray dried CSP7 mix containing 25% leucine was analyzed by modulated differential scanning calorimetry. Pictured is the mDSC curve.
**FIG. 25****: Thermal properties of Spray Dried CSP7 with Trehalose.** Spray dried CSP7 mix containing 25% trehalose was analyzed by modulated differential scanning calorimetry. Pictured is the mDSC curve.
**FIG. 26****: Thermal properties of Spray Dried CSP7 with Sodium Citrate.** Spray dried CSP7 mix containing 25% sodium citrate was analyzed by modulated differential scanning calorimetry. Pictured is the mDSC curve.
**FIG. 27****: Thermal properties of Spray Dried CSP7 with Leucine and Trehalose.** Spray dried CSP7 mix containing 15% leucine and 10% trehalose was analyzed by modulated differential scanning calorimetry. Pictured is the mDSC curve.
**FIG. 28****: Thermal properties of All Spray Dried CSP7 Mixtures.** Each of the manufactured spray dried CSP7 mixtures was analyzed by mDSC. All mDSC curves for these powders are pictured.
**FIG. 29****: Wet Weight of Dissected Lung Tissues from Mice.** Euthanized mice were dissected and their lungs removed and weighed. Mice were either saline treated, bleomycin treated to induce lung fibrosis, or bleomycin treated and treated with CSP7 peptide for 12 or 60 min.
**FIG. 30****: Collagen Content of Murine Lung Tissue.** Untreated, bleomycin treated, or bleomycin and CSP7 treated lung tissues were homogenized and collagen content was analyzed using the Quickzyme collagen assay. Graph depicts total collagen content of the lungs.
**FIG. 31****: Ashcroft Scores of Murine Lung Tissues.** Untreated, bleomycin treated, or bleomycin and CSP7 treated lung tissues were homogenized and collagen content was analyzed using the Quickzyme collagen assay. Ashcroft scores were determined as described by Hubner et al. 2008. Graph depicts total collagen content of the lungs.
**FIG. 32****: Collagen Content of Murine Lung Tissue.** Untreated, bleomycin treated, or bleomycin and CSP7 treated lung tissues were homogenized and collagen content was analyzed using the Quickzyme collagen assay. Graph depicts total collagen content of the lungs.
**FIG. 33****:** Stability of CSP7 (ammonium counterion) following up to 5 freeze-thaw cycles.
**FIG. 34****:** Specific surface area of milled and neat CSP7 (ammonium counterion) powder.
**FIG. 35****:** Thermogravimetric Analysis of milled and neat CSP7 (ammonium counterion) powder.
**FIG. 36****:** SEM images of milled and neat CSP7 (ammonium counterion) powder.
**FIG. 37****:** Appearance of milled CSP7 (ammonium counterion) powder in stability study.
**FIG. 38****:** Crystallinity of milled CSP7 (ammonium counterion) in the stability study.

### DESCRIPTION OF ILLUSTRATIVE EMBODIMENTS

### I. Definitions

As used herein, "essentially free," in terms of a specified component, is used herein to mean that none of the specified component has been purposefully formulated into a composition and/or is present only as a contaminant or in trace amounts. The total amount of the specified component resulting from any unintended contamination of a composition is therefore well below 0.01%. Most preferred is a composition in which no amount of the specified component can be detected with standard analytical methods.

As used herein the specification, "a" or "an" may mean one or more. As used herein in the claim(s), when used in conjunction with the word "comprising," the words "a" or "an" may mean one or more than one.

The use of the term "or" in the claims is used to mean "and/or" unless explicitly indicated to refer to alternatives only or the alternatives are mutually exclusive, although the disclosure supports a definition that refers to only alternatives and "and/or." As used herein "another" may mean at least a second or more.

The term "peptide" as used herein typically refers to a sequence of amino acids made up of a single chain of amino acids joined by peptide bonds. Generally, peptides contain at least two amino acid residues and are less than 50 amino acids in length, unless otherwise defined. A peptide may be provided with a counterion. Likewise, in some cases, a peptide may include a N and/or C-terminal modification such a as blocking modification that reduced degradation.

A "biologically active" caveolin-1 (Cav-1) peptide refers to a peptide that increases p53 protein levels, reduces urokinase plasminogen activator (uPA) and uPA receptor (uPAR), and/or increases plasminogen activator inhibitor-1 (PAI-1) expression in cells, such as fibrotic lung fibroblasts. In the present invention, the biologically active peptide is the amino acid sequence of SEQ ID NO: 2.

The term "identity" or "homology" shall be construed to mean the percentage of amino acid residues in the candidate sequence that are identical with the residue of a corresponding sequence to which it is compared, after aligning the sequences and introducing gaps, if necessary to achieve the maximum percent identity for the entire sequence, and not considering any conservative substitutions as part of the sequence identity. Neither N- or C-terminal extensions nor insertions shall be construed as reducing identity or homology. Methods and computer programs for the alignment are well known in the art. Sequence identity may be measured using sequence analysis software.

The term "polypeptide" or "protein" is used in its broadest sense to refer to a compound of two or more subunit amino acids, amino acid analogs, or peptidomimetics. The subunits may be linked by amide bonds. In another embodiment, the subunit may be linked by other bonds, e.g. ester, ether, etc. As used herein the term "amino acid" refers to either natural and/or unnatural or synthetic amino acids, including glycine and both the D or L optical isomers, and amino acid analogs and peptidomimetics. The term "peptidomimetic" or "peptide mimic" means that a peptide is modified in such a way that it includes at least one non-peptidic bond such as, for example, urea bond, carbamate bond, sulfonamide bond, hydrazine bond, or any other covalent bond. A peptide of three or more amino acids is commonly called an oligopeptide if the peptide chain is short. If the peptide chain is long (*e.g.,* longer than 50 amino acids), the peptide is commonly called a polypeptide or a protein.

The terms "subject" and "individual" and "patient" are used interchangeably herein, and refer to an animal, for example a human or non-human animal (*e.g.,* a mammal), to whom treatment, including prophylactic treatment, with a pharmaceutical composition for use as disclosed herein, is provided. The term "subject" as used herein refers to human and non-human animals. The term "non-human animals" includes all vertebrates, *e.g*., mammals, such as non-human primates, (particularly higher primates), sheep, dogs, rodents (*e.g*. mouse or rat), guinea pigs, goats, pigs, cats, rabbits, cows, and non-mammals such as chickens, amphibians, reptiles etc. In one embodiment, the subject is human. In another embodiment, the subject is an experimental animal or animal substitute as a disease model. Non-human mammals include mammals such as non-human primates, (particularly higher primates), sheep, dogs, rodents (*e.g.* mouse or rat), guinea pigs, goats, pigs, cats, rabbits and cows. The non-human animal may be a companion animal such as a dog or a cat.

"Treating" a disease or condition in a subject or "treating" a patient having a disease or condition refers to subjecting the individual to a pharmaceutical treatment, e.g., the administration of a drug, such that at least one symptom of the disease or condition is decreased or stabilized., As the peptide is administered therapeutically as a treatment of an inflammatory disorder or fibrotic condition of the lungs, it is typically administered to a subject who presents with one or more symptoms of lung injury or lung fibrosis.

By "isolated" it is meant that the polypeptide has been separated from any natural environment, such as a body fluid, *e.g.,* blood, and separated from the components that naturally accompany a polypeptide.

By isolated and "substantially pure" is meant a polypeptide that has been separated and purified to at least some degree from the components that naturally accompany it. Typically, a polypeptide is substantially pure when it is at least 60%, or at least 70%, at least 80%, at least 90%, at least 95%, or even at least 99%, by weight, free from the proteins and naturally-occurring organic molecules with which it is naturally associated. For example, a substantially pure polypeptide may be obtained by extraction from a natural source, by expression of a recombinant nucleic acid in a cell that does not normally express that protein, or by chemical synthesis.

By "covalently bonded" is meant j oined either directly or indirectly (*e.g*., through a linker) by a covalent chemical bond. For example, the fusion peptides are covalently bonded.

The term "fusion protein" as used herein refers to a recombinant protein of two or more proteins. Fusion proteins can be produced, for example, by a nucleic acid sequence encoding one protein is joined to the nucleic acid encoding another protein such that they constitute a single open-reading frame that can be translated in the cells into a single polypeptide harboring all the intended proteins. The order of arrangement of the proteins can vary. Fusion proteins can include an epitope tag or a half-life extender. Epitope tags include biotin, FLAG tag, c-myc, hemaglutinin, His6, digoxigenin, FITC, Cy3, Cy5, green fluorescent protein, V5 epitope tags, GST, β-galactosidase, AU1, AU5, and avidin. Half-life extenders include Fc domain and serum albumin.

The term "airway" refers herein to any portion of the respiratory tract including the upper respiratory tract, the respiratory airway, and the lungs. The upper respiratory tract includes the nose and nasal passages, mouth, and throat. The respiratory airway includes the larynx, trachea, bronchi and bronchioles. The lungs include the respiratory bronchioles, alveolar ducts, alveolar sacs and alveoli.

The terms "inhalational smoke induced acute lung injury" and "ISALI" are used interchangeably herein and refer to a form of acute lung injury (ALI) caused by smoke inhalation. ALI is also referred to as "mild Acute Respiratory Distress Syndrome; ARDS." ARDS can be defined by finding one or more of the following conditions in a subject: 1) bilateral pulmonary infiltrates on chest x-ray, 2) when measured by right heart catheterization as clinically indicated, pulmonary capillary wedge pressure < 18 mmHg (2.4 kPa), and 3) PaO₂/FiO₂ <300 mmHg (40 kPa). Treatment of ISALI may include treatment of one or more of the following conditions: reduced oxygenation, airway obstruction (including a severe airway obstruction), fibrinous airway casts or debris, and alveolar fibrin deposition.

The term "air jet mill" refers to a device or method for reducing particle size by using a jet of compressed gas to impact particles into one another, thereby pulverizing the particles. An air jet mill may be used to reduce the size of peptide particles. Other mechanical milling devices that perform the same function can also be used interchangeably with the air jet mill. Air jet milling can occur under various environmental parameters such as temperature, pressure, relative/absolution humidity, oxygen content, etc.

The term "ball mill" refers to a device or method for reducing particle size by adding the particle of interest and a grinding medium to the interior of a cylinder and rotating the cylinder. The particles of interest are broken down as the grinding medium rises and falls along the exterior of the cylinder as it rotates. A ball mill may be used to reduce the size of peptide particles. Other mechanical milling devices that perform the same function can also be used interchangeably with the air jet mill.

The term "wet mill" or "media mill" refers to a device or method for reducing particle size by adding the particle of interest to device with an agitator, containing a media comprising a liquid and a grinding medium. With the addition of the particle of interest, as the agitator rotates, the energy it disperses causes the grinding medium and particles of interest to come into contact and break down the particles of interest. Other mechanical milling devices that perform the same function can also be used interchangeably with the air jet mill.

The term "high pressure homogenization" refers to a method of reducing particle size by adding the particle of interest to a device which combines both pressure and mechanical forces to break down the particle of interest. Mechanical forces used in high pressure homogenization may include impact, shear, and cavitation, among others. Other mechanical milling devices that perform the same function can also be used interchangeably with the air jet mill.

The term "cryogenic mill" refers to a device or method for reducing particle size by first chilling a particle of interest with dry ice, liquid nitrogen, or other cryogenic liquid, and subsequently milling the particle of interest to reduce the size. Other mechanical milling devices that perform the same function can also be used interchangeably with the air jet mill.

The phrase "effective amount" or "therapeutically effective" means a dosage of a drug or agent sufficient to produce a desired therapeutic result. The desired therapeutic result can be subjective or objective improvement in the recipient of the dosage, reduced infection, reduced inflammation, increased lung growth, increased lung repair, reduced tissue edema, increased DNA repair, decreased apoptosis, a decrease in tumor size, a decrease in the rate of growth of cancer cells, a decrease in metastasis, or any combination of the above.

As used herein, "excipient" refers to pharmaceutically acceptable carriers that are relatively inert substances used to facilitate administration or delivery of an Active Pharmaceutical Ingredient (API) into a subject or used to facilitate processing of an API into drug formulations that can be used pharmaceutically for delivery to the site of action in a subject. Excipients or pharmaceutically acceptable carriers include all of the inactive components of the dosage form except for the active ingredient(s).. The pharmaceutical composition for use of the present invention is excipient free. This means that it is free of any excipients.

The phrases "pharmaceutical composition" or "pharmacologically acceptable composition" refers to molecular entities and compositions that do not produce an adverse, allergic, or other untoward reaction when administered to an animal, such as a human, as appropriate. The present invention concerns a pharmaceutical composition for use in the treatment of an inflammatory disorder or fibrotic condition of the lungs, the pharmaceutical composition for use comprising a dry powder of a peptide, or a pharmaceutically acceptable salt thereof, wherein the peptide is the amino acid sequence of SEQ ID NO: 2; wherein the pharmaceutical composition for use is in the form of a dry powder formulation and to be administered using a dry powder inhaler, and the pharmaceutical composition for use is excipient free. Moreover, for animal (*e.g*., human) administration, it will be understood that preparations should meet bioburden, sterility, pyrogenicity, general safety, and/or purity standards as required by the FDA or other recognized regulatory authority.

The term "pharmaceutically acceptable carrier" includes any and all excipients, processing aids, aqueous solvents (*e.g*., water, alcoholic/aqueous solutions, saline solutions, parenteral vehicles, such as sodium chloride, Ringer's dextrose, etc.), non-aqueous solvents (*e.g*., propylene glycol, polyethylene glycol, vegetable oil, and injectable organic esters, such as ethyloleate), dispersion media, coatings, surfactants, antioxidants, preservatives (*e.g*., antibacterial or antifungal agents, anti-oxidants, chelating agents, and inert gases), isotonic agents, absorption delaying agents, salts, drugs, drug stabilizers, gels, binders, disintegration agents, lubricants, flavor modifiers (*e.g*., sweetening agents, flavoring agents), such like materials and combinations thereof, as would be known to one of ordinary skill in the art. The pH and exact concentration of the various components in the pharmaceutical composition for use are adjusted according to well-known parameters. The above definition of the "pharmaceutically acceptable carrier" is merely given for reference, as it is emphasized that the pharmaceutical composition for use of the present invention is excipient free.

### II. The Caveolin-1 derived peptide of SEQ ID NO: 2

The present invention concerns a pharmaceutical composition for use in the treatment of an inflammatory disorder or fibrotic condition of the lungs, the pharmaceutical composition for use comprising a dry powder of a peptide, or a pharmaceutically acceptable salt thereof, wherein the peptide is the amino acid sequence of SEQ ID NO: 2; wherein the pharmaceutical composition for use is in the form of a dry powder formulation and to be administered using a dry powder inhaler, and the pharmaceutical composition for use is excipient free. The amino acid sequence of SEQ ID NO: 2 is a caveolin-1 (Cav-1) scaffolding domain or a peptide that interferes with Cav-1 interaction with Src kinases mimics the combined effect of uPA and anti-β1-integrin antibody. Native human Cav-1 has a length of 178 amino acids and a molecular weight of 22 kDa. The amino acid sequence of Cav-1 is shown below (SEQ ID NO: 1).

1 MSGGKYVDSE GHLYTVPIRE QGNIYKPNNK AMADELSEKQ VYDAHTKEID LVNRDPKHLN
61 DDVVKIDFED VIAEPEGTHS FDGIWKASFT TFTVTKYWFY RLLSALFGIP MALIWGIYFA
121 ILSFLHIWAV VPCIKSFLIE IQCISRVYSI YVHTVCDPLF EAVGKIFSNV RINLQKEI

The following features of the peptides are in accordance with the present invention in as far as they apply to the amino acid sequence of SEQ ID NO: 2. Thus, in the following table only SEQ ID NO: 2 is according to the present invention.

**Table 1: Exemplary Cav-1 peptides.**

| **Sequence** | **ID** |
|---|---|
| FTTFTVT | SEQ ID NO: 2 |
| ASFTTFTVT | SEQ ID NO: 3 |
| KASFTTFTVTKGS | SEQ ID NO: 4 |
| KASFTTFTVTKGS-NH2 | SEQ ID NO: 5 |
| aaEGKASFTTFTVTKGSaa | SEQ ID NO: 6 |
| aaEGKASFTTFTVTKGSaa-NH2 | SEQ ID NO: 7 |
| Ac-aaEGKASFTTFTVTKGSaa-NH2 | SEQ ID NO: 8 |
| OASFTTFTVTOS | SEQ ID NO: 9 |
| OASFTTFTVTOS-NH2 | SEQ ID NO: 10 |
| FTTFTVT-NH2 | SEQ ID NO: 11 |
| FTTFTVTK-NH2 | SEQ ID NO: 12 |
| KASFTTFTVTK-NH2 | SEQ ID NO: 13 |
| Ac-KASFTTFTVTK-NH2 | SEQ ID NO: 14 |
| OASFTTFTVTK-NH2 | SEQ ID NO: 15 |
| Ac-OASFTTFTVTK-NH2 | SEQ ID NO: 16 |
| Ac-KASFTTFTVTKGS-NH2 | SEQ ID NO: 17 |
| DSGKASFTTFTVTK-NH2 | SEQ ID NO: 18 |
| Ac-DSGKASFTTFTVTK-NH2 | SEQ ID NO: 19 |
| Ac-OASFTTFTVTOS-NH2 | SEQ ID NO: 20 |
| H-DGIWKASF-NH2 | SEQ ID NO: 21 |
| H-VTKYWFYR-NH2 | SEQ ID NO: 22 |

| | |
|---|---|
| (a=D-Alanine, O=Omithine) | |

The peptides in the above table are biologically active derivatives which have the activity of the native Cav-1 polypeptide in *in vitro* or *in vivo* assays of binding or of biological activity. In the present invention, the peptide is of the amino acid sequence of SEQ ID NO: 2 with the following sequence: FTTFTVT: The peptide may inhibit or prevent apoptosis of lung epithelial cells (LECs) induced by bleomycin *in vitro* or *in vivo* with activity at least 20% of the activity of the native Cav-1 polypeptide, or at least 30%, 40%, 50%, 60 %, 65%, 70%, 75%, 80%, 85%, 90%, 95%, 97%, 99%, and any range derivable therein, such as, for example, from 70% to 80%, and more preferably from 81% to 90%; or even more preferably, from 91% to 99%. The peptide may have 100% or even greater activity than the native CAV-1 polypeptide. Assays for testing biological activity, *e.g*., anti-fibrotic activity, the ability to affect expression of uPA, uPAR and PAI-1 mRNAs, or inhibit proliferation of lung fibroblasts, are well-known in the art.

### A. Pharmaceutical Compositions For Use

The present invention concerns a pharmaceutical composition for use in the treatment of an inflammatory disorder or fibrotic condition of the lungs, the pharmaceutical composition for use comprising a dry powder of a peptide, or a pharmaceutically acceptable salt thereof, wherein the peptide is the amino acid sequence of SEQ ID NO: 2; wherein the pharmaceutical composition for use is in the form of a dry powder formulation and to be administered using a dry powder inhaler, and the pharmaceutical composition for use is excipient free. The following considerations are thus only applicable to the present invention in as far as they fulfill this requirement.

The dry powder of the peptide of the amino acid sequence of SEQ ID NO: 2 may be to be administered in combination, simultaneously or sequentially with at least one additional therapeutic (e.g., a therapeutic for treatment of lung fibrosis). The additional therapeutic may be an NSAID, steroid, DMARD, immunosuppressive, biologic response modulators, bronchodilator or antifibrotic agent such as pirfenedone, an agent whose antifibrotic mechanism of action is not fully understood but may involve blockade of TGF-beta, nintedanib, a broad tyrosine kinase blocker or any other antifibrotic agent. Suitable NSAIDS are selected from the non-selective COX-inhibitors acetylsalicyclic acid, mesalazin, ibuprofen, naproxen, flurbiprofen, fenoprofen, fenbufen, ketoprofen, indoprofen, pirprofen, carprofen, oxaprozin, pranoprofen, miroprofen, tioxaprofen, suprofen, alminoprofen, tiaprofenic acid, fluprofen, indomethacin, sulindac, tolmetin, zomepirac, nabumetone, diclofenac, fenclofenac, alclofenac, bromfenac, ibufenac, aceclofenac, acemetacin, fentiazac, clidanac, etodolac, oxpinac, mefenamic acid, meclofenamic acid, flufenamic acid, nifluminic acid, tolfenamic acid, diflunisal, flufenisal, piroxicam, tenoxicam, lornoxicam and nimesulide and the pharmaceutically acceptable salts thereof, the selective COX 2-inhibitors meloxicam, celecoxib and rofecoxib and the pharmaceutically acceptable salts thereof. Suitable steroids are prednisone, prednisolone, methylprednisolone, dexamethasone, budenoside, fluocortolone and triamcinolone. Suitable DMARDs are sulfasalazine, olsalazine, chloroquin, gold derivatives (Auranofin), D-penicillamine and cytostatics such as methotrexate and cyclophosphamide. Suitable immunsuppressives are cyclosporine A and derivatives thereof, mycophenolatemofetil, FK 506, OKT-3, ATG, 15-desoxyspergualin, mizoribine, misoprostol, rapamycin, reflunomide and azathioprine. Suitable biologic response modifiers are interferon β, anti-TNF-α (Etanercept), IL-10, anti-CD3 or anti-CD25. Suitable bronchodilators are ipratropium bromide, oxytropiumbromide, tiotropiumbromide, epinephrinehydrochloride, salbutamole, terbutalinsulfate, fenoterolhydrobromide, salmeterole and formoterole. In such combinations each active ingredient can be administered (e.g., orally or by inhalation) either in accordance with its usual dosage range or a dose below its usual dosage range. The dosage for the combined NSAIDs, steroids, DMARDs, immunsuppressives and biologic response modifiers is appropriately 1/50 of the lowest dose normally recommended up to 1/1 of the normally recommended dosage, preferably 1/20 to 1/2 and more preferably 1/10 to 1/5. The normally recommended dose for the combined drug should be understood to be the dose disclosed for example in Rote Liste® 2002, Editio Cantor Verlag Aulendorf, Germany, or in Physician's Desk Reference.

Where clinical applications are contemplated, it may be necessary to prepare pharmaceutical compositions for use comprising proteins, antibodies, and drugs in a form appropriate for the intended application. The phrases "pharmaceutical or pharmacologically acceptable" refers to molecular entities and compositions that do not produce an adverse, allergic, or other untoward reaction when administered to an animal, such as, for example, a human, as appropriate. It is emphasized that the pharmaceutical composition for use of the present invention, as defined in claim 1, is excipient free . Moreover, for animal (e.g., human) administration, it will be understood that preparations should meet bioburden, sterility, pyrogenicity, general safety, and/or purity standards as required by the FDA Office of Biological Standards or other appropriate regulatory authority.

Information on ways of administration can be found in Remington's Pharmaceutical Sciences, 18th Ed., 1990). The choice of injection volume and needle size may be chosen by the person of ordinary skill in the art based on site of injection, syringeability and injectability, which includes considering the viscosity of the solution or suspension to be injected and drug concentration, pH, and osmolality. In some instances, the particle size of the active agent can be chosen in order to provide a desired rate of dissolution upon administration (e.g., by subcutaneous injection).

The peptide of the amino acid sequence of SEQ ID NO: 2 may be formulated in a free base, neutral, zwitterion or salt form. Pharmaceutically acceptable salts include the acid addition salts, *e.g*., those formed with the free amino groups of a proteinaceous composition, or which are formed with inorganic acids, such as, for example, hydrochloric or phosphoric acids, or such organic acids as acetic, oxalic, tartaric, or mandelic acid. Salts formed with the free carboxyl groups can also be derived from inorganic bases, such as, for example, sodium, potassium, ammonium, calcium, or ferric hydroxides; or such organic bases as isopropylamine, trimethylamine, histidine, or procaine.

The therapeutic may be administered to the patient in a single dose or in multiple doses. When multiple doses are administered, the doses may be separated from one another by, for example, one hour, three hours, six hours, eight hours, one day, two days, one week, two weeks, or one month. For example, the therapeutic may be administered for, *e.g.,* 2, 3, 4, 5, 6, 7, 8, 10, 15, 20, or more weeks. It is to be understood that, for any particular subject, specific dosage regimes should be adjusted over time according to the individual need and the professional judgment of the person administering or supervising the administration of the composition for use. For example, the dosage of the therapeutic can be increased if the lower dose does not provide sufficient therapeutic activity.

While the attending physician ultimately will decide the appropriate amount and dosage regimen, therapeutically effective amounts of the one or more polypeptides as disclosed herein or a mutant, variant, analog or derivative thereof may be provided at a dose of 0.0001, 0.01, 0.01 0.1, 1, 5, 10, 25, 50, 100, 500, or 1,000 mg/kg or g/kg. Effective doses may be extrapolated from dose-response curves derived from in vitro or animal model test bioassays or systems.

Dosages for a particular patient or subject can be determined by one of ordinary skill in the art using conventional considerations, (e.g., by means of an appropriate, conventional pharmacological protocol). A physician may, for example, prescribe a relatively low dose at first, subsequently increasing the dose until an appropriate response is obtained. The dose administered to a patient is sufficient to affect a beneficial therapeutic response in the patient over time, or, *e.g.,* to reduce symptoms, or other appropriate activity, depending on the application. The dose is determined by the efficacy of the particular formulation, and the activity, stability or serum half- life of the peptide of the amino acid sequence of SEQ ID NO: 2 and the condition of the patient, as well as the body weight or surface area of the patient to be treated.

A subject to be treated with the pharmaceutical composition for use of the present invention may be given a single dose, given once daily for treating a subject, preferably a mammal, more preferably human who his suffering from or susceptible to pulmonary fibrosis resulting therefrom is between 0.2 mg/kg and 250 mg/kg, such as between 10 mg/kg and 50 mg/kg, for example, via instillation (by inhalation). Such a dose can be administered daily for anywhere from 3 days to one or more weeks. Chronic administration is also possible, though the dose may need to be adjusted downward as is well-understood in the art. The foregoing ranges are, however, suggestive, as the number of variables in an individual treatment regime is large, and considerable excursions from these preferred values are expected.

### B. Dry Powder Particle Size Reduction and Dry Powder Inhalation Devices.

The particle sizes of the formulations can be reduced by any suitable method, including but not limited to milling, grinding, thin film freezing, spray drying, or crushing. Milling may be performed by any method known in the art, such as by air jet mill, ball mill, wet mill, media mill, high pressure homogenization, or cryogenic mill.

Peptide stability following particle size reduction can be assessed using known techniques in the art, including size exclusion chromatography; electrophoretic techniques; HPLC; mass spectrometry; spectroscopic techniques such as UV spectroscopy and circular dichroism spectroscopy, and activity (measured *in vitro* or *in vivo*). To perform *in vitro* assays of protein stability, an aerosol composition can be collected and then distilled or absorbed onto a filter. To perform *in vivo* assays, or for pulmonary administration of a composition to a subject, a device for dry powder dispersion is adapted for inhalation by the subject. For example, protein stability can be assessed by determining the level of protein aggregation. Preferably, a dry powder composition of the invention is substantially free of protein aggregates. The presence of soluble aggregates can be determined qualitatively using dynamic light scattering (DLS) (DynaPro-801TC, Protein Solutions Inc. of Charlottesville, Va.) and/or by UV spectrophotometry.

An inhaler may be a passive dry powder inhaler (DPI), such as a Plastiape RSO1 monodose DPI. In a dry powder inhaler, dry powder is stored in a reservoir and is delivered to the lungs by inhalation without the use of propellants.

The pharmaceutical composition for use of the present invention is a pharmaceutical composition for use in the treatment of an inflammatory disorder or fibrotic condition of the lungs, wherein the pharmaceutical composition for use comprises a dry powder of a peptide, or a pharmaceutically acceptable salt thereof, wherein the peptide is the amino acid sequence of SEQ ID NO: 2; wherein the pharmaceutical composition for use is in the form of a dry powder formulation and to be administered using a dry powder inhaler, and the pharmaceutical composition for use is excipient free.. An example of an inhaler is a single-dose DPI, such as a DoseOne^{™}, Spinhaler, Rotohaler^{®}, Aerolizer^{®}, or Handihaler. In some embodiments, an inhaler is a multidose DPI, such as a Plastiape RS02, Turbuhaler^{®}, Twisthaler^{™}, Diskhaler^{®}, Diskus^{®}, or Ellipta^{™}. In some embodiments, an inhaler is a plurimonodose DPI for the concurrent delivery of single doses of multiple medications, such as a Plastiape RS04 plurimonodose DPI. Typically, dry powder inhalers have medication stored in an internal reservoir, and medication is delivered by inhalation with or without the use of propellants. Dry powder inhalers may require an inspiratory flow rate greater than 30 L/min for effective delivery, such as between 30-120 L/min. In some embodiments, efficient aerosolization of milled CSP7 is independent of inspiratory force. In some embodiments, the dry powder inhaler has a flow resistance of between 0.01 kPa^{0.5}min/L and 0.05 kPa^{0.5}min/L, such as between 0.02 kPa^{0.5}min/L and 0.04 kPa^{0.5}min/L. The dry powder inhaler (e.g., high resistance, low resistance, passive, active) is chosen based on the patient population and their inspiratory capabilities.

The composition for use may be administered on a routine schedule. As used herein, a routine schedule refers to a predetermined designated period of time. The routine schedule may encompass periods of time which are identical or which differ in length, as long as the schedule is predetermined. For instance, the routine schedule may involve administration twice a day, every day, every two days, every three days, every four days, every five days, every six days, a weekly basis, a monthly basis or any set number of days or weeks there-between. *A*lternatively, the predetermined routine schedule may involve administration on a twice daily basis for the first week, followed by a daily basis for several months, *etc.* In some embodiments, the peptide of the amino acid sequence of SEQ ID NO: 2 is to be administered once per day. The peptide in the composition for use may be administered less than once per day, such as every other day, every third day, or once per week. In some embodiments, a complete dose of the peptide is between 1-100 mg, such as 20-100, 50-100, 10-20, 20-40, 50-70, or 80-90 mg.

The peptide of the amino acid sequence of SEQ ID NO: 2 may be provided in a unit dosage form (*e.g*., pre-divided dose), such as in a capsule, blister or a cartridge, wherein the unit dose comprises at least 1mg of a peptide, such as at least 5 mg, 10 mg, 15 mg or 20 mg of a peptide of the embodiments (*e.g.,* CSP7) per dose. The unit dose may be 1-10 mg (*e.g.,* 5 mg) of the peptide. The unit dosage form does not comprise the administration or addition of any excipient and is merely used to hold the powder for inhalation (i.e., the capsule, blister, or cartridge is not administered). More than one of the unit dose forms may be administered to a subject. The peptide of the amino acid sequence of SEQ ID NO: 2 may be provided in unit dose capsules and more than one unit dose capsules (e.g., 3-4) can be administered to a subject by inhalation. The peptide of the amino acid sequence of SEQ ID NO: 2 may be administered in a high emitted dose, such as at least 10 mg, preferably at least 15 mg, even more preferably 20 mg. Administration of the peptide of the amino acid sequence of SEQ ID NO: 2may result in a high fine particle dose into the deep lung such as greater than 5 mg. Preferably, the fine particle dose into the deep lung is at least 10 mg, even more preferably at least 15 mg. The particle dose may be produced from 1, 2, 3, 4 or 5 or more capsules comprising doses of the peptide of the amino acid sequence of SEQ ID NO: 2. It is emphasized that the pharmaceutical composition for use of the present invention is excipient freeThe fine particle dose may be at least, 50%, such as at least 60, 65, 70, 75, or 80% of the emitted dose.

Changes in inhalation pressure drop may result in a change in emitted dose. In some embodiments, changes in inhalation pressure of 3 kPa, such as from 4 kPa to 1 kPa, result in a reduction of emitted dose of less than 25%, such as 24%, 23%, 22%, 21%, 20%, 19%, 18%, 17%, 16%, 15%, 14%, 13%, 12%, 11%, 10%, 9%, 8%, 7%, 6%, 5% or less. Changes in inhalation pressure may result in a change in fine particle dose. Changes in inhalation pressure of 3 kPa, such as from 4kPa to 1 kPa may result in a reduction of fine particle dose of less than 15%, such as 14%, 13%, 12%, 11%, 10%, 9%, 8%, 7%, 6%, 5% or less.

### IV. Pulmonary conditions for treatment

The peptide of the amino acid sequence of SEQ ID NO: 2 as comprised in the pharmaceutical composition for use can be used to treat a variety of pulmonary conditions. Pulmonary conditions for treatment may be acute or chronic. Acute pulmonary conditions may be acute lung injury, infection or chemical-induced. Chronic pulmonary conditions maybe the result of injury, infection or disease.

The pharmaceutical composition for use of the present invention is for use in the treatment of an inflammatory disorder or fibrotic condition of the lungs. The following considerations regarding conditions to be treated are thus only applicable to the present invention in as far as the treatment of an inflammatory disorder or fibrotic condition of the lungs is concerned.

### A. Lung injuries

The subject to be treated in accordance with this invention may have an acute lung injury (ALI) or infection or a chemical-induced lung injury. The subject to be treated in accordance with this invention may have subject has plastic bronchitis, asthma, chronic obstructive airway/pulmonary (COPD), acute respiratory distress syndrome (ARDS) inhalational smoke induced acute lung injury (ISALI), bronchiectasis, inhalational toxin-induced airway disease (e.g., chlorine or other induced airways disease), exposure to mustard gas, exposure to particulate matter (e.g., silica dust), bronchiolitis obliterans, bronchiolitis obliterans organizing pneumonia, collagen vascular lung disease (e.g., from lupus, scleroderma or mixed connective tissue disease), interstitial lung disease (e.g., idiopathic pulmonary fibrosis or sarcoidosis), drug induced lung disease and accelerated pulmonary fibrosis (e.g., that occurs after acute lung injury including ARDS). Lung diseases including chronic obstructive pulmonary disease, asthma, infections, as well as acute and chronic lung injury leading to fibrosis, constitute the third leading cause of death world-wide (Murray et al., 1997; Rabe et al., 2007; Tsushima et al., 2009). Acute lung injury (ALI) is a serious medical problem amongst American military personnel. ALI during combat can result from very broad etiologies.

ALI from inhalational injury has been treated with inhaled anticoagulants, steroids, beta-agonists, high frequency ventilation, and extra-corporeal membrane oxygenation, with variable and, in general, suboptimal results. No effective preventive measures are available other than barriers with respiratory masks. The management of ARDS has progressed significantly but remains largely supportive with watchful waiting for endogenous healing mechanisms to take effect; and in-hospital mortality remains above 40% (Matthay et al., 2012). Survivors of ALI often suffer chronic respiratory disability with reduced quality of life. Any modalities that can accelerate recovery and/or prevent later complications such as chronic respiratory insufficiency and pulmonary fibrosis will be highly desirable. There is a dire need to improve the early diagnosis and much more importantly, prevention and therapy of ALI. The pathophysiology of ALI from direct inhalational lung injury or ARDS consequent to systemic illness is extremely complex and heterogeneous, encompassing systemic as well as local cardiopulmonary factors such as increased membrane permeability, influx of inflammatory cytokines, oxidative cellular damage, compartmental fluid shifts, deranged ion channels, and many others (Matthay et al., 2012). Clearly, novel treatments are needed for treating and preventing lung disorders such as ALI.

### B. Lung diseases

Lung diseases include pulmonary fibrosis, pulmonary inflammation, idiopathic pulmonary fibrosis, cystic fibrosis, chronic obstructive pulmonary disease (COPD), bronchitis, bronchiolitis, bronchiolitis obliterans, asthma, and pulmonary infections, as well as acute and chronic lung injury leading to fibrosis (Murray *et al.,* 1997; Rabe *et al.,* 2007; Tsushima *et al.,* 2009). These diseases constitute the third leading cause of death world-wide.

Cystic fibrosis is an inherited disease of the exocrine glands and exocrine sweat glands which primarily affects the digestive and respiratory systems. This disease usually characterized by chronic respiratory infections, pancreatic insufficiency, abnormally viscid mucous secretions and premature death. Cystic fibrosis (CF) is characterized by progressive airflow obstruction. Subsets of individuals with CF also develop airway hyper-responsiveness to inhaled cholinergic agonists (Weinberger, 2002 and Mitchell *et al.,* 1978) and reversibility of airflow limitation in response to bronchodilators (van Haren *et al.,* 1991 and van Haren *et al.,* 1992). The presence of bronchial hyper-responsiveness and airway obstruction suggest a possible shared etiology of disease between CF and other diseases of airway narrowing such as asthma or chronic obstructive pulmonary disease (COPD) where airway smooth muscle dysfunction is thought to contribute to the disease processes.

A pulmonary infection may be a bacterial infection. The infectious bacteria may be *Pseudomonas aeruginosa, Bacillus anthracis, Listeria monocytogenes, Staphylococcus aureus, Salmenellosis, Yersina pestis, Mycobacterium leprae, M. africanum, M. asiaticum, M. aviuin-intracellulaire, M. chelonei abscessus, M. fallax, M. fortuitum, M. kansasii, M. leprae, M. malmoense, M. shimoidei, M. simiae, M. szulgai, M. xenopi, M. tuberculosis, Brucella melitensis, Brucella suis, Brucella abortus, Brucella canis, Legionella pneumonophilia, Francisella tularensis, Pneurnocystis carinii, mycoplasma, or Burkholderia cepacia.* The bacterial infection may result in pneumonia.

Chronic obstructive pulmonary disease (COPD) is a term used to classify two major airflow obstruction disorders: chronic bronchitis and emphysema. Approximately 16 million Americans have COPD, 80-90% of them were smokers throughout much of their lives. COPD is a leading cause of death in the U.S., accounting for 122,283 deaths in 2003. The cost to the USA for COPD was approximately $20.9 billion in direct health care expenditures in 2003. Chronic bronchitis is inflammation of the bronchial airways. The bronchial airways connect the trachea with the lungs. When inflamed, the bronchial tubes secrete mucus, causing a chronic cough.

In emphysema, the alveolar sacs are overinflated as a result of damage to the elastin skeleton of the lung. Inflammatory cells in emphysematous lung release elastase enzymes, which degrade or damage elastin fibers within the lung matrix. Emphysema has a number of causes, including smoking, exposure to environmental pollutants, alpha-one antitrypsin deficiency, and aging.

Bronchiolitis is most commonly caused by viral lower respiratory tract infections, and primarily characterized by acute inflammation, edema, necrosis of epithelial cells lining small airways, and increased mucus production (Ralston *et al.,* 2014). Signs and symptoms typically begin with rhinitis and cough, which may progress to tachypnea, wheezing, rales, use of accessory muscles, and/or nasal flaring.

Bronchiolitis obliterans is a progressive airflow reduction as a result of abnormal remodeling of the small airways in the lungs (Meyer *et al.,* 2014). Bronchiolitis obliterans syndrome is a major complication of lung transplantations and is often used to describe a delayed allograft dysfunction that results in persistent decline in forced expiratory volume and force that is not caused by other known causes (Meyer *et al.,* 2014).

The term "asthma" may refer to acute asthma, chronic asthma, intermittent asthma, mild persistent asthma, moderate persistent asthma, severe persistent asthma, chronic persistent asthma, mild to moderate asthma, mild to moderate persistent asthma, mild to moderate chronic persistent asthma, allergic (extrinsic) asthma, non-allergic (intrinsic) asthma, nocturnal asthma, bronchial asthma, exercise induced asthma, occupational asthma, seasonal asthma, silent asthma, gastroesophageal asthma, idiopathic asthma and cough variant asthma. During asthma, the airways are persistently inflamed and may occasionally spasm.

### V. Examples

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result within the scope of the invention, which is as defined in the claims. Several experimental examples relate to the preparation of a pharmaceutical composition for use according to the present invention. It is, however to be understood that the present invention relates not to the pharmaceutical composition as a product, but only to the pharmaceutical composition for use in the treatment of an inflammatory disorder or fibrotic condition of the lungs, the pharmaceutical composition for use comprising a dry powder of a peptide, or a pharmaceutically acceptable salt thereof, wherein the peptide is the amino acid sequence of SEQ ID NO: 2; wherein the pharmaceutical composition for use is in the form of a dry powder formulation and to be administered using a dry powder inhaler, and the pharmaceutical composition for use is excipient free.

### Example 1 - Methods and Materials

*Preparation of dry powder peptides.* CSP7 peptide (SEQ ID NO: 2), lot #: AHF66//470103 was synthesized by Polypeptide Laboratories (San Diego, USA).

*Manufacture of CSP7 mixes and spray drying.* CSP7 formulations containing either CSP7 alone (CSP7), or a 75%/25% mix of CSP7/Leucine, CSP7/Trehalose, or CSP7/Sodium Citrate, or a 75%/15%/10% mix of CSP7/Leucine/Trehalose were prepared in pH 10 water (adjusted with NH₄OH) and spray dried using a BLD-35 with 2" cyclone.

*Reduction of dry powder CSP7 particle size by Thin Film Freezing (TFF). 0.3* mg/ml CSP7 bulk powder and 0.9 mg/ml Mannitol (mass ratio 1:3) were dissolved in 10 mM Tris buffer, and the pH was adjusted to 8.05. The solution was then filtered with a 0.45 µm membrane and dropped onto a rolling chamber filled with liquid nitrogen. The metered freezing temperature was between -55 and -65°C. The frozen flakes were then lyophilized in a VirTis AdVantage Freeze Dryer (VirTis Company Inc., NY, US). The lyophilization conditions were as follows: equilibration: hold at -55 °C, 100 mTorr for 30 min; primary drying: ramp temperature up to -30 °C, 100 mTorr over 250 min; hold at -30 °C, 100 mTorr for 660 min; secondary drying: ramp to 30 °C, 100 mTorr over 720 min; and hold at 30 °C, 100 mTorr for 240 min. The TFF processed sample is referred to as batch 171014.

*Reduction of dry powder CSP7 particle size by Cryogenic Milling.* One gram of CSP7 bulk powder is added into a small cryomill tube and then loaded in 6870 Freeze/Mill (SPEX Certiprep^{™}, NJ, USA). Milling was done in 5 cycles, a 10 minute precool, and each cycle having a 5 minute run time at 10 CPS followed by a cooling of 2 minutes. Milled sample was retrieved and weighed, and yield was calculated to be 73.5% based on retrieved weight over loaded weight.

*Reducing particle size of CSP7powder by Ball Mill (BM).* CSP7 bulk powder was suspended in its antisolvent, ethanol (anhydrous), to a concentration of 1 mg/ml. Approximately half of the solvent volume of Zirconium balls (2 mm) were added to the suspension. The suspension was then loaded into an 8000M Mixer/Mill (SPEX SamplePrep, NJ, USA) to mill. Samples were collected from the milling process to test at 5min, 10 min, and 30 min, respectively.

*Reduction of particle size of CSP7 powder using a Rotor-stator.* CSP7 bulk powder was dispersed into ethanol to a concentration of 1 mg/ml. The tip (5 mm*75 mm flat bottom) of the Rotor-stator was submerged in the suspension and homogenization was performed to reduce the particle size.

*High Performance Liquid Chromatography Analysis.* Samples were dissolved in 20 mM Tris buffer (pH 10.3), and run on a Phenomenex Luna^{®} C18(2) liquid chromatography column with 5 µm particle size and 100 Å pore size. The Phenomenex Security Guard Cartridge Kit was used as a guard column. Mobile phase A was H₂O + 0.1% (v/v) Trifluoroacetic acid (TFA), and mobile phase B was 80% acetonitrile + 20% H₂O + 0.09% (v/v) TFA. The sample was injected in a volume of 20 µL. The samples were run for 25 min each, and the column was kept at 25 °C, at a flow rate of 1 ml/min. The samples were detected at a wavelength of 220 nm. The buffer gradient was set to the conditions specified in Table 2.

| **Table 2. Materials, conditions, and methods for HPLC** | | | |
|---|---|---|---|
| **Separation Column** | Phenomenex Luna^{®} 5 µm C18(2) 100 Å, LC Column 150 x 4.6 mm | | |
| **Guard Column** | Phenomenex Security Guard Catridge Kit | | |
| **Mobil Phase A** | H₂O + 0.1% Trifluoroacetic acid (TFA) | | |
| **Mobil Phase B** | 80% Acetonitrile + 20% H₂O + 0.09% TFA | | |
| **Injection Volume** | 20 µL | | |
| **Run Time** | 25 min | | |
| **Column Temp.** | 25 °C | | |
| **Flow** | 1 ml/min | | |
| **Wavelength** | 220 nm | | |

| | Time (min) | A% | B% |
|---|---|---|---|
| **Gradient** | 0 | 75 | 25 |
| | 20 | 65 | 35 |
| | 21 | 75 | 25 |
| | 25 | 75 | 25 |

*Determination of aerodynamic particle size distribution of milled CSP7 bulk powder.* Around 3.5 mg of milled CSP7 powder was manually filled into size 3 HPMC capsules (Capsugel, Peapack, NJ). CSP7 capsules were then aerosolized using a RS01 Monodose Dry Powder Inhaler (high resistance) and aerodynamic particle size distribution was measured by a Next Generation Impactor (NGI, MSP Corp., Shoreview, MN). Aerosols were produced over 4 s at an air flow rate of 60 L/min for the inhaler to achieve an inhalation volume of 4 L and 4kPa pressure drop across the device. Before each run, NGI collection surfaces were coated with 5% (v/v) polysorbate 20 in methanol. One capsule was shot for each run and each sample was ran in triplicate repetitions (three capsules). After aerosolization, all collection surfaces were rinsed with specific volumes of 20 mM Tris buffer (pH 10.3) to collect the drug. Powders deposited in the capsule, device, adaptor, throat, pre-separator, and stages 1-MOC were extracted respectively.

For each test, delivered dose is defined as the mass of CSP7 that entered the NGI. Geometric standard deviation (GSD), mass median aerodynamic diameter (MMAD) and fine particle fraction% (FPF%) are calculated and analyzed with Copley Inhaler Testing Data Analysis Software (CITDAS, Copley Scientific, Nottingham UK), based on the doses deposited on stages 1-MOC of NGI. The FPF is defined as the mass fraction of particles less than 5.0 µm with the delivered dose.

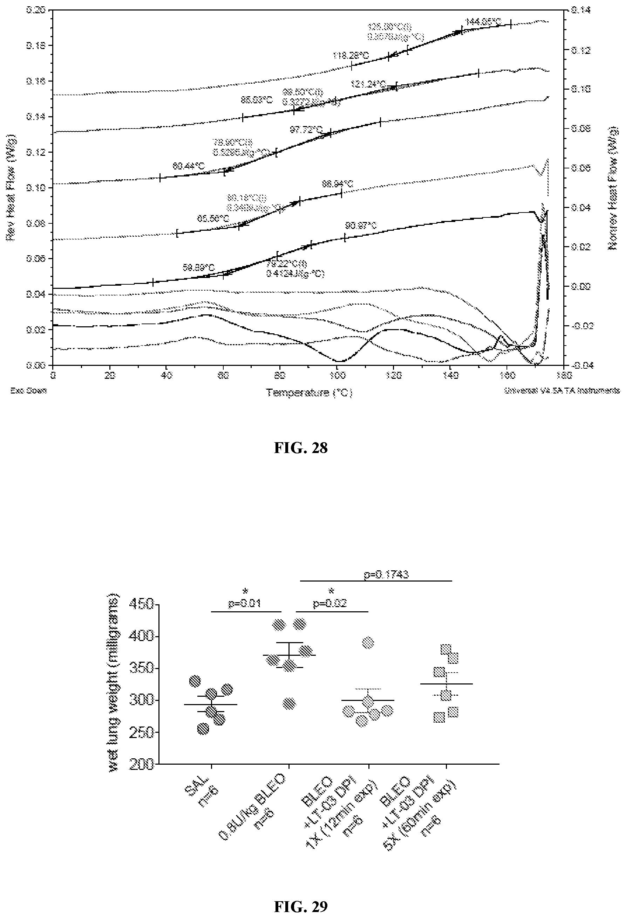

*Preparation and lysis of lung tissues.* Female mice, ages 6-8 weeks are ordered from Jackson Laboratories, stock: 000664 C57BL/6J are cared for and housed according to IACUC guidelines. The following week, mice are weighed, anesthetized with an IP injection of 80mg/kg Ketamine & 6 mg/kg Xylazine (approx. 115ul/mouse) and subjected to a single intratracheal installation of bleomycin. Briefly, a 26G plastic catheter is inserted into the trachea and mice receive, via pipette, 2 x 20ul installations (spaced 30s apart to allow clearance from the airways) of 0.8U/kg bleomycin (Biotang, Cat# RB003). Controls receive the same volume of saline only. Weight is tracked (approximately 10% weight loss occurs in injured animals) and animals are subjected to a dry powder inhalation protocol (CH technologies) daily for one week. The dry powder dosing was based on the minimum efficacious dose of previous nebulization formulation which was estimated to be a lung delivered dose of 0.7mcg/animal (Tepper *et al.,* 2016). In summary, an exposure time of 12min/day matched a '1X' dose and a '5X' dose designated a 60min/day treatment equivalent to 3.5mcg/animal. Animals were exposed for seven consecutive days (d14-20; during the fibrotic phase of bleomycin injury) and sacrificed 24hrs following the last dose lethal dose of heparinized ketamine/xyalizine cocktail (25% heparin). A subset of whole lungs were harvested for histology. Briefly, a transcardiac perfusion with 10mls of saline clears the blood from the lung. Then, the lungs were inflated with saline for 1min, followed by 4%PFA for Imin from 20cm above the dissection stage. The trachea was tied off and the lung was excised, fixed, embedded and sectioned to 4 microns to achieve visualization of the maximum surface area, and stained with hematoxylin and eosin. Images were captured with Aperio AT2 high volume, digital whole slide scanner and lungs were scored for fibrotic injury according to modified Ashcroft scoring protocol (Hübner et al., 2008). For molecular analyses, whole lungs were homogenized in RIPA buffer and protease inhibitors (Santa Cruz) as well as 1% DTT (to inhibit RNAses), homogenized in parallel at 4C (Precellys Evolution, Bertin Instruments) and processed for downstream assays. Collagen content of lung homogenate was assayed according using Total Collagen Assay (Quickzyme) using the collagen standard provided by the manufacturer and according to manufacturer's instructions. The colormetric assay was read on a microplate reader (FilterMax F5, Molecular Devices, 580nm). In addition, RNA was extracted from homogenate (Zymogen Research) and reverse transcribed to cDNA (Qiagen, QuantiNova Reverse Transcription 205413). Results of these studies are show in FIGs. 29-32.

Homogenization buffer for 28 samples was prepared by adding 224 µL of cocktail inhibitor, 224 µL of NaOV4, 224 µL of PMSF, and 0.22 g of DTT to 22.4 mL of RIPA buffer. 800 µL of homogenization buffer was added per sample. Samples were homogenized using the Precellys Evolution with CK28 beads. The homogenization protocol used was for hard tissue and performed twice at 4 °C for each sample. The homogenized samples were then aliquoted out, with 400 µL stored for BCA concentration determination and protein assays, 200 µL for RNA isolation, and 200 µL for the collagen assay.

*Collagen assay.* Collagen standard was prepared for use by adding 125 µL of the Quickzyme collagen standard to 125 µL of 12 M HCL, and 200 µL of each sample to 200 µL of 12 M HCl. The samples and standard were incubated at 95 °C for 20 hours, and vortex briefly after 20 minutes. Following incubation, samples were centrifuged for 10 minutes at 13,000 x g. The standard was prepared according to the manufacturer's instructions (Quickzyme). 100 µL of each sample was then diluted into 50 µL of water. 10 µL of each diluted sample was then further diluted into 100 µL of 4M HCl. Duplicates of the standards and each of the samples were pipetted into plates. 75 µL of assay buffer was added to each well, and the plate was covered before shaking for 20 minutes. 75 µL of detection reagent mix was added per well, and the plate was mixed before incubating at 60 °C for one hour. Plates were then read as indicated above.

*RNA isolation.* RNA isolation was performed using the Qiagen RNeasy kit, according to the manufacturer's instructions. Briefly 25 µL of RLT buffer and 75 µL of 70% ethanol were added to 50 µL of sample in RIPA buffer, yielding a total of 150 µL of starting material. 50 µL of the starting material for each sample was then added to 50 µL of RNase free water. Then, 350 µL of buffer RLT was added, and the samples were mixed well. 250 µL of 95-100% ethanol were then added to each, and the samples were mixed again. 700 µL of each sample were then added to their respective spine columns and centrifuged at 8000 X g, and the flowthrough was discarded. 500 µL of RPE was added to each column, and the columns were centrifuged again. 500 µL of RPE was added again, and this time the samples were centrifuged for 2 min at 8000 x g. Samples were transferred to a new microcentrifuge tube, and RNA was eluted with 40 µL of RNase free water by centrifugation for 1 min at 8000 x g. Samples were quantified by nanodrop and analyzed as described above.

### Example 2 - Characterization of CSP7 Bulk Powder

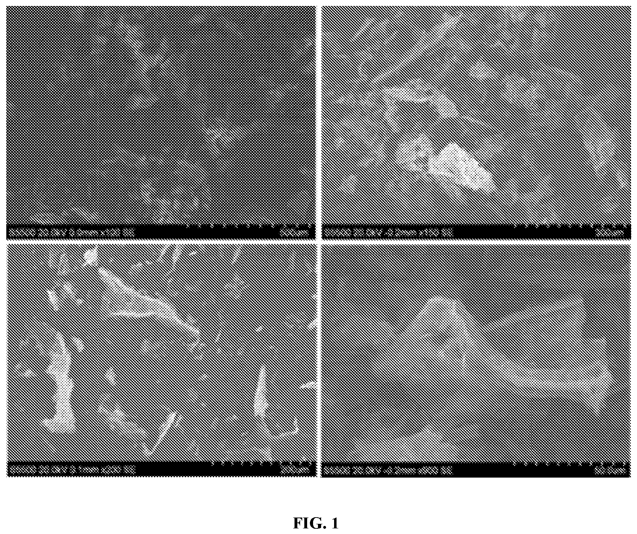

*Scanning Electron Microscopy.* Bulk powder samples of CSP7 were sputtered on the sample tray and spread by blowing compressed nitrogen. Samples were imaged by scanning electron microscopy (FIG. 1). SEM indicates the existence of large (>5 µm) particles. Further, most of the particles appeared to be large (>5 µm), and thus out of the respirable range.

*CSP7 Particle Size Evaluation.* Particle sizes were examined using Spraytec laser diffraction and Sympatec laser diffraction instrument HELOS-R system, equipped with solid or wet dispersion attachment, to determine whether bulk powder is within the respirable range. The size of CSP7 bulk powder particles was determined to be larger than the respirable particle size using the dry dispersion method. Table 3 shows the particle sizes for the particles evaluated to be at Dv 10, Dv 50 (median), and Dv 90 within the distribution. As can be seen in Table 3, greater than 50% of all CSP7 particles analyzed had a particle size of 5.3 µm or larger, which is larger than the respirable range.

| **Table 3: Particle size of CSP7 bulk powder by Sympatec laser diffraction dry dispersion** | | |
|---|---|---|
| **Dv 10 (µm)** | **Dv 50 (µm)** | **Dv 90 (µm)** |
| 1.4±0.0 | 5.3±0.0 | 22.0±1.7 |

The particle sizes were next determined by Sympatec laser diffraction by a wet dispersion method. CSP7 was dissolved in ethanol + 0.05% Tween 80 as dispersion medium and sonicated 10 min. The results of the size determination of CSP7 particles using wet dispersion are shown in Table 4. As shown in Table 4, the average particle size (Dv 50) of the wet dispersion particles was 29.0 ± 0.8, far outside of the respirable range.

| **Table 4: Particle size of CSP7 bulk powder by Sympatec laser diffraction wet dispersion** | | |
|---|---|---|
| **Dv 10 (µm)** | **Dv 50 (µm)** | **Dv 90 (µm)** |
| 7.2±0.1 | 29.0±0.8 | 63.9±3.8 |

Average particle size was further evaluated using the Spraytec laser diffraction instrument, again using a dry dispersion method. The CSP7 bulk powder was dispersed at 40 PSI, and again the average particle size (8.6±1.5 µm) was above the respirable range (Table 5).

| **Table 5: Particle size of CSP7 bulk powder by Spraytec laser diffraction dry dispersion** | | | |
|---|---|---|---|
| **Dv 10 (µm)** | **Dv 50 (µm)** | **Dv 90 (µm)** | **% V < 5 µm %** |
| 1.7±0.2 | 29.0±0.8 | 63.9±3.8 | 34.5±4.1 |

The percentage of the dry powder particles found to be smaller than 5 µm was found to be only 34.5 ± 4.1%. Given that each of the laser diffraction methods found the majority of the bulk powder to have a particle size outside the respirable range, any dry powder used for treatment would need to be processed in some way to reduce particle size.

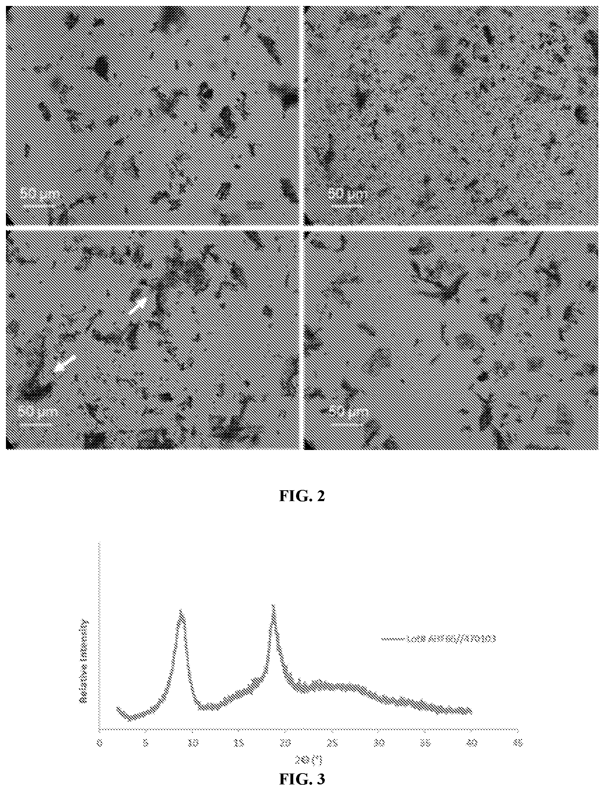

*Morphology of Bulk CSP7 powder.* Bulk powder CSP7 samples were sputtered onto glass slides and viewed by optical microscopy (FIG. 2). Optical microscopy confirmed the SEM, indicating the presence of large (>5 µm) particles. Optical microscopy also shows the presence of particle agglomerates, visible in FIG. 2 by arrows.

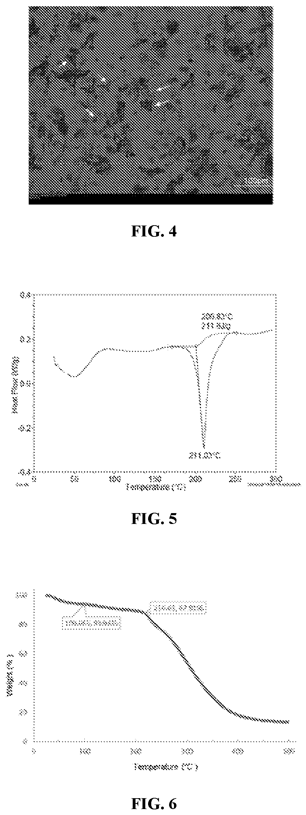

*Determination of Crystallinity of CSP7 Bulk Powder Particles.* CSP7 bulk powder particles were evaluated using X-ray powder diffraction (FIG. 3). It was found that neat CSP7 exhibits some crystallinity by X-ray powder diffraction (FIG. 3). To confirm the X-ray diffraction results, crystallinity was evaluated by polarized light microscopy (FIG. 4). As can be seen in FIG. 4, there is crystalline CSP7 present in the bulk CSP7 powder, and certain typical crystalline forms are indicated by white arrows in the image.

*Thermo-analysis of CSP7 Bulk Powder.* Differential Scanning Calorimetry was used to determine the melting point of neat CSP7 (FIG. 5). As determined by DSC, the melting point of CSP7 was determined to be 211.03 °C (FIG. 5). Analysis was continued using Thermogravimetric analysis (TGA) (FIG. 6). The TGA indicates that the weight of neat CSP7 starts dramatically decreasing above 216 °C (FIG. 6).

*Moisture Content of CSP7 Bulk Powder.* The moisture content of bulk powder CSP7 was evaluated by Karl Fischer-Volumetric (KF-V) titration using a Mettler Toledo Karl Fischer Titrator, and performed in triplicate. Table 6 shows the moisture content of each test, and the average moisture content from the 3 tests.

| **Table 6: Moisture content of CSP7 bulk powder.** | |
|---|---|
| **Test** | **Moisture Content** |
| 1 | 2.9% |
| 2 | 3.0% |
| 3 | 3.1% |
| **Average** | **3.0%** |

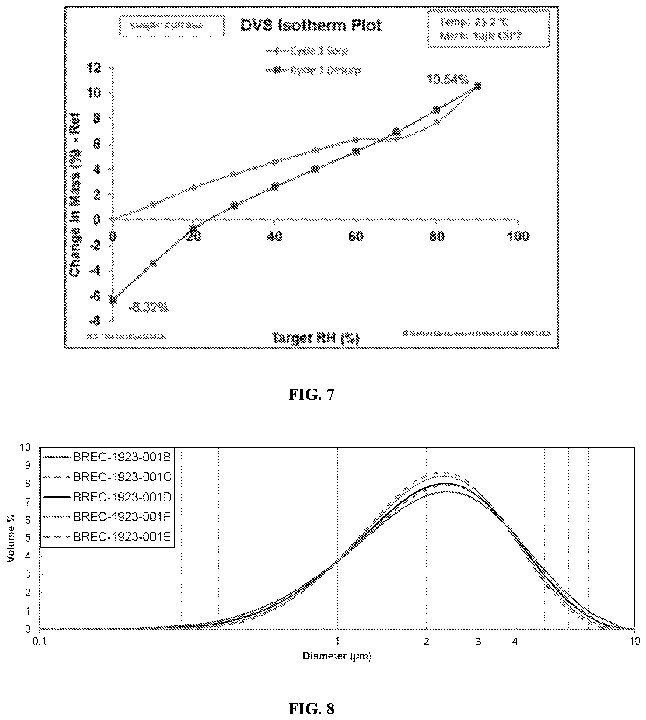

Next, the moisture sorption of CSP7 bulk powder was analyzed using dynamic vapor sorption, DVS (FIG. 7). The CSP7 sample was run for a full sorption/desorption cycle, and the sample was found to have a 6.32% moisture desorption in relative humidity of zero. The mass change was found to be 10.54% when the relative humidity is 90% (FIG. 7).

### Example 3 - Characterization of CSP7 Powders Following Particle Size Reduction

*Particle Size Reduction of CSP7Powder.* In order to effectively inhale and deposit a powder into the lungs, the particle size should generally have a mass median aerodynamic diameter of less than 5 µm. A variety of techniques were performed to reduce particle size of the neat material, including air jet milling (AJM), ball milling (BM), cryogenic milling (CM), thin film freezing (TFF), and spray drying. First, AJM was performed to reduce the particle size of the CSP7 bulk powder, and the milled CSP7 was collected from several locations within the mill. Yield and particle size distribution of a first batch of milled powder (Batch 171013) collected from the indicated positions are shown in Tables 5 and 6. Particle size distribution was determined by Sympatec laser diffraction dry dispersion (Table 8), or Sympatec laser diffraction wet dispersion (Table 9), as above.

| **Table 8: Particle size distribution and yield of CSP7 powder (Batch 171013) using dry dispersion** | | | | | |
|---|---|---|---|---|---|
| **Position** | **Dv 10 (µm)** | **Dv 50 (µm)** | **Dv 90 (µm)** | **% 1-5 µm** | **Yield %** |
| BFC+ Grinding Chamber | 1.2 ± 0.0 | 2.7 ± 0.1 | 5.1 ± 0.2 | 83.7 ± 1.3 | 18% |
| Cyclone | 1.0± 0.0 | 2.7 ± 0.2 | 5.7 ± 0.0 | 73.6 ± 0.1 | 9% |
| Vessel Adapter | 1.0 ± 0.0 | 2.5 ± 0.0 | 4.9 ± 0.1 | 82.3 ± 0.8 | 6% |
| Vessel | 1.1 ± 0.0 | 2.5 ± 0.1 | 4.9 ± 0.1 | 82.6 ± 1.4 | 10% |

| **Table 9: Particle size distribution and yield of CSP7 powder (Batch 171013) using wet dispersion** | | | | | |
|---|---|---|---|---|---|
| **Position** | **Dv 10 (µm)** | **Dv 50 (µm)** | **Dv 90 (µm)** | **% 1-5 µm** | **Yield %** |
| BFC+ Grinding Chamber | 1.2 ± 0.0 | 3.1 ± 0.0 | 6.4 ± 0.1 | 80.0 ± 11.2 | 18% |
| Cyclone | 1.0± 0.0 | 3.4 ± 0.1 | 7.4 ± 0.2 | 61.3 ± 1.0 | 9% |
| Vessel Adapter | 1.1 ± 0.0 | 3.0 ± 0.0 | 5.9 ± 0.1 | 75.3 ± 0.9 | 6% |
| Vessel | 1.2 ± 0.0 | 2.9 ± 0.0 | 5.8 ± 0.0 | 78.2 ± 0.6 | 10% |

The second batch (Batch 171027) of CSP7 was milled from 10 grams of neat bulk powder using the same conditions as above. Again, powder size distribution and yield were assessed from the same locations and are listed in Table 10.

| **Table 10: Particle size distribution and yield of CSP7 powder (Batch 171027) using dry dispersion** | | | | | |
|---|---|---|---|---|---|
| **Position** | **Dv 10 (µm)** | **Dv 50 (µm)** | **Dv 90 (µm)** | **% 1-5 µm** | **Yield%** |
| Bag+bag adaptor | 0.7±0.0 | 1.4±0.0 | 3.2±0.1 | 68.3±0.6 | 19.0 |
| BFC+ Grinding Chamber | 1.0±0.0 | 2.6±0.1 | 5.0±0.2 | 81.0±1.3 | 16.9 |
| Cyclone | 0.9±0.0 | 2.7±0.1 | 5.6±0.1 | 73.1±1.2 | 17.2 |
| Vessel Adaptor | 0.9±0.0 | 2.3±0.0 | 4.7±0.1 | 80.3±1.2 | 7.3 |
| collection vessel | 0.9±0.0 | 2.3±0.0 | 4.4±0.1 | 82.2±1.2 | 15.9 |
| Total | | | | | 76.3 |

A third batch of CSP7 dry powder was subjected to thin film freezing (TFF) (batch 171014) and analyzed as above, using both Sympatec Laser Diffraction Dry Dispersion (Table 11) and Wet Dispersion (Table 12).

| **Table 11: Particle size distribution and yield of CSP7 powder (Batch 171014) using dry dispersion** | | | |
|---|---|---|---|
| **Dv 10 (µm)** | **Dv 50 (µm)** | **Dv 90 (µm)** | **% 1-5 µm** |
| 2.1 ± 0.0 | 5.7 ± 0.1 | 10.4 ± 0.3 | 37.0 ± 0.6 |

| **Table 12: Particle size distribution and yield of CSP7 powder (Batch 171014) using wet dispersion** | | | |
|---|---|---|---|
| **Dv 10 (µm)** | **Dv 50 (µm)** | **Dv 90 (µm)** | **% 1-5 µm** |
| 3.8 ± 0.0 | 7.1 ± 0.0 | 13.3 ± 0.2 | 24.6 ± 0.7 |

Another batch of CSP7 dry powder was subjected to cryogenic milling (CM) to reduce the particle size. The particle size of the CM CSP7 was assessed by laser diffraction using dry dispersion (Table 13) and wet dispersion (Table 14) as above.

| **Table 13: Particle size distribution and yield of CM CSP7 powder (dry dispersion)** | | | |
|---|---|---|---|
| **Dv 10 (µm)** | **Dv 50 (µm)** | **Dv 90 (µm)** | **% 1-5 µm** |
| 0.7 ± 0.0 | 2.7 ± 0.2 | 17.4 ± 0.9 | 42.7 ± 0.5 |

| **Table 14: Particle size distribution and yield of CM CSP7 powder (wet dispersion)** | | | |
|---|---|---|---|
| **Dv 10 (µm)** | **Dv 50 (µm)** | **Dv 90 (µm)** | **% 1-5 µm** |
| 1.5 ± 0.0 | 10.5 ± 0.1 | 38.5 ± 0.7 | 28.2 ± 0.1 |

Another batch of CSP7 bulk powder was subjected to ball milling (BM) to reduce the particle size. The particle size distributions of BM CSP7 powder taken from several time points during the milling process are presented in Table 15.

| **Table 15: Particle size distribution and yield of BM CSP7 powder (wet dispersion)** | | | | |
|---|---|---|---|---|
| **Duration** | **Dv 10 (µm)** | **Dv 50 (µm)** | **Dv 90 (µm)** | **% 1-5 µm** |
| 5 min | 0.8 ± 0.0 | 3.8 ± 0.2 | 12.5 ± 0.4 | 43.0 ± 0.7 |
| 10 min | 0.7 ± 0.0 | 2.3 ± 0.1 | 9.2 ± 0.2 | **49.0 ±** 0.6 |
| 30 min | 0.6 ± 0.0 | 1.4 ± 0.0 | 5.3 ± 0.3 | 55.5 ± 0.7 |

Further batches were produced with mixtures of CSP7 (also referred to by the assigned development acronym, CSP7) and leucine, trehalose, sodium citrate, or leucine and trehalose, and subjected to spray drying to reduce particle size. The size of spray dried particles was examined by dry dispersion laser diffraction (Fig. 8, Table 16). Again, relative to Bulk CSP7, spray drying significantly reduced the size of the CSP7 particles.

| **Table 16: Spray dry manufacturing of CSP7 mixtures** | | | | | |
|---|---|---|---|---|---|
| **Lot ID** | **BREC-1923-001B** | **BREC-1923-001C** | **BREC-1923-001D** | **BREC-1923-001E** | **BREC-1923-001F** |
| **Formulation** | 100% | 75/25 | 75/25 | 75/25 | 75/15/10 |
| | CSP7 | CSP7/Leucine | CSP7/Trehalose | CSP7/Sodium Citrate | CSP7/Leucine/Tre halose |
| **Dryer** | BLD-35 with 2' cyclone | | | | |
| **Solvent** | pH = ~10 water (adjusted with NH₄OH) | | | | |
| **Nozzle** | 2-Fluid | | | | |
| **Batch Size (g)** | 4 | 4 | 4 | 2 | 4 |
| **Mass Yield (g)** | 3.07 | 2.98 | 3.12 | 1.60 | 3.04 |
| **% Yield** | 77 | 75 | 78 | 79 | 76 |
| **D(v,0.1) (µm)** | 0.81 ±0.04 | 0.91 ±0.00 | 0.85 ±0.04 | 0.84 ±0.04 | 0.89 ±0.01 |
| **D(v,0.5) (µm)** | 1.99±0.01 | 1.96 ±0.00 | 1.98 ±0.02 | 1.99 ±0.01 | 1.97 ±0.02 |
| **D(v,0.9) (µm)** | 4.20 ±0.12 | 3.79±0.01 | 4.01 ±0.10 | 4.06 ±0.11 | 3.88 ±0.03 |

CSP7 particle size was dramatically reduced following each of the methods for reducing the particle size (air jet milling, thin film freezing, cryogenic milling, ball milling, and spray drying), both when measured by solid dispersion, and wet dispersion (Compare Tables 1 and 2 to Tables 5, 6, 10, 13, 14, 16, 17, 18 and 19). Following air jet milling, the majority of the CSP7 particles fall within the respirable range, however thin film freezing, cryogenic milling, and ball milling were not quite as effective, with smaller percentages of the milled powders falling within the respirable range when measured by laser diffraction using solid dispersion or liquid dispersion.

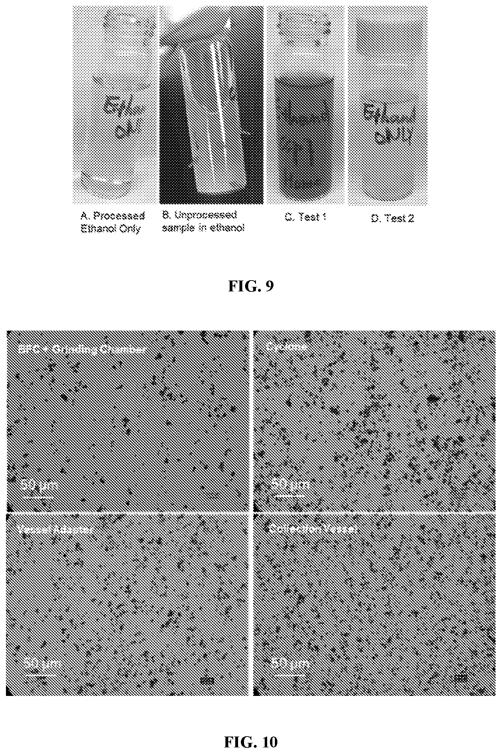

Particle size was also reduced using a rotor-stator handheld homogenizer (FIG. 9). CSP7 changed color in ethanol following homogenization to either light or dark grey, depending on the duration and power of homogenization FIG. 9, and it is clear that homogenization reduced particle size, though the method was not further pursued due to the observed color change.

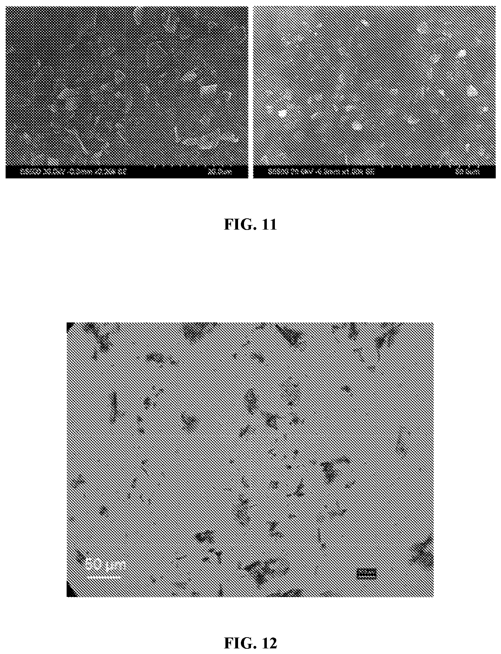

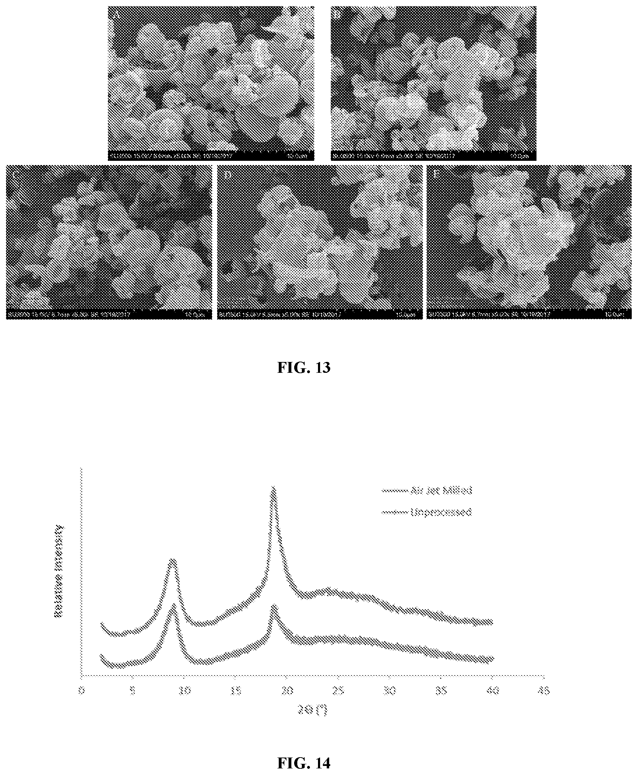

*Morphology of milled CSP7 powders.* Morphology of each of the milled samples was examined using optical microscopy or Scanning Electron Microscopy. The powder samples from the air jet mill were examined as above and the optical microscopy indicated that the particle size was reduced to a size of 1 µm > particle size > 5µm, and homogenous (FIG. 10). Further, the milled CSP7 particles were free of agglomerates (FIG. 10). SEM indicated that the homogenized and reduced particle size of the air jet milled particles was between 1 and 5 µm (FIG. 11). The powder samples obtained after TFF were also examined, and while particle sizes were larger, the sample was found to be free of agglomerate (FIG. 12). Further analysis included assessment of the particle morphology of the spray dried formulations by scanning electron microscopy. Representative SEM images of the formulations are shown in FIG. 13.

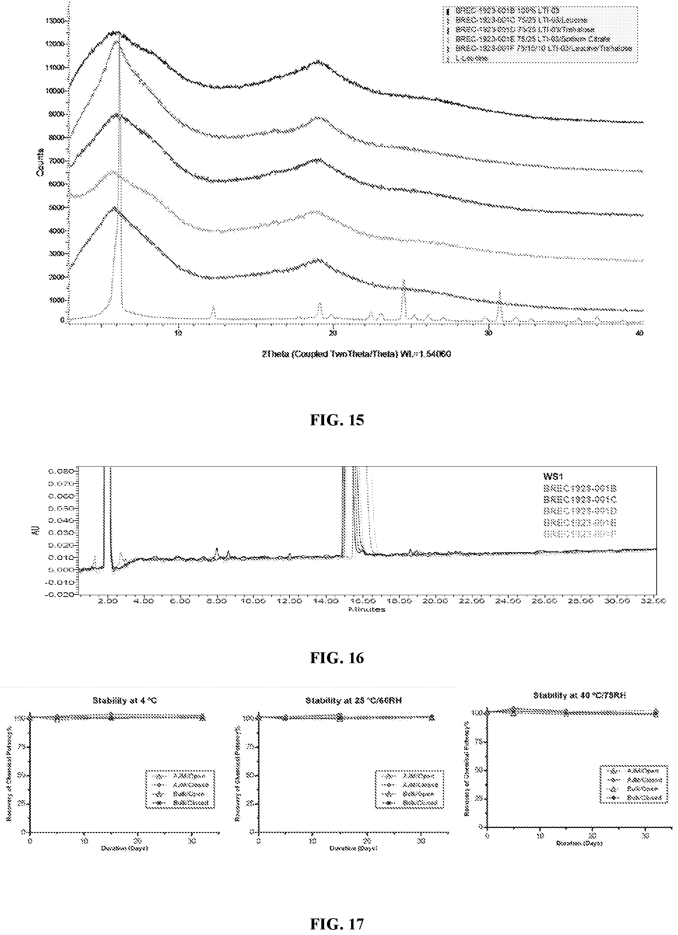

*Crystallinity of AJM CSP7.* Milled CSP7 powder (batch 171027) was evaluated by X-ray diffraction and crystallinity exhibited in diffractogram (FIG. 14). Crystallinity of spray dried CSP7 was also examined by X-ray diffraction and the curves are pictured in FIG. 15. The formulations of CSP7 alone, or CSP7 coupled with trehalose or sodium citrate are amorphous, while CSP7 coupled with leucine or leucine and trehalose appear to contain crystalline leucine character, as shown by the sharp peaks in FIG. 15.

*HPLC Evaluation of Milled or Spray Dried CSP7 Powder.* To determine whether milling the CSP7 bulk powder had any effect on chemical potency, samples of milled CSP7 powder were collected from various parts of the mill were assessed using HPLC performed under the conditions listed in Table 2. Potency was evaluated using the equation: $Potency = \frac{Free CSP 7 peptide conc , from HPLC result}{Free CSP 7 peptide conc . from mass balance} \times 100 %$ It is clear from Table 26, that milling had no deleterious effect on the potency of any of the samples collected. Similarly, when Batch 171027 was tested, the chemical potency was determined to be 100.14%

| **Table 26: Potency of milled CSP7 powder (Batch 171013)** | | | | |
|---|---|---|---|---|
| **Position** | **Collection Vessel** | **Vessel Adaptor** | **Cyclone** | **BCF + Chamber** |
| Potency by HPLC | 100.7% | 101.3% | 102.6% | 100.7% |

The spray dried CSP7 mixtures was examined by RP-HPLC to examine purity (Table 17, FIG. 16). Similar to the air jet milled CSP7 powder, the spray dried CSP7 retains roughly 100% purity.

| **Table 17: Purity of spray dried formulations of CSP7** | | | | | | | | | |
|---|---|---|---|---|---|---|---|---|---|
| Relative Abundance >LOQ | | | Impurity #1 | Impurity #2 | Impurity #3 | **Primary ACTIVE Peak** | Impurity #4 | Impurity #5 | Total Impurities |
| Retention Time | | | 7.99 | 8.62 | 12.03 | 14.93 | 18.30 | 18.86 | |
| Relative Ret. Time | | | 0.54 | 0.58 | 0.81 | 1.00 | 1.23 | 1.26 | |
| API standard | 1/0/00 | | 0.60 | 0.26 | - | 99.01 | --- | 0.13 | 0.99 |
| API standard | 1/0/00 | | 0.27 | 0.26 | 0.14 | 99.16 | --- | 0.16 | 0.84 |
| 100% CSP7 | BREC1923-001 B | A1 | --- | --- | --- | 100.00 | --- | --- | --- |
| 100% CSP7 | BREC1923-001B | A2 | --- | --- | --- | 99.64 | --- | 0.36 | 0.36 |
| 75/25 CSP7/Leucine | BREC1923-001C | B1 | --- | --- | --- | 100.00 | --- | --- | --- |
| 75/25 CSP7/Leucine | BREC1923-001C | B2 | --- | --- | --- | 99.79 | --- | 0.21 | 0.21 |
| 75/25 CSP7/Trehalose | BREC1923-001D | C1 | --- | --- | --- | 99.63 | 0.14 | 0.23 | 0.37 |
| 75/25 CSP7/Trehalose | BREC1923-001D | C2 | --- | --- | --- | 99.53 | 0.16 | 0.31 | 0.47 |
| 75/25 CSP7/ Sodium Citrate | BREC1923-001 E | D1 | --- | --- | --- | 99.72 | 0.11 | 0.17 | 0.28 |
| 75/25 CSP7/ Sodium Citrate | BREC1923-001 E | D2 | --- | --- | --- | 99.82 | 0.18 | --- | 0.18 |
| 75/15/10 CSP7/ Leucine/Trehalose | BREC1923-001F | E1 | --- | --- | --- | 99.84 | 0.16 | --- | 0.16 |
| 75/15/10 CSP7/ Leucine/Trehalose | BREC1923-001F | E2 | --- | --- | --- | 99.84 | 0.16 | --- | 0.16 |

*CSP7 stability.* The stability of both unprocessed bulk CSP7 powder, as well as air jet milled CSP7 (batch 171027) were examined by assaying their chemical potency using HPLC. A sample of each was stored in 3 different conditions before assaying the chemical potency at 5, 15, and 32 days of storage (FIG. 17). Stability of spray dried CSP7 over a period of 24 hours was also examined by HPLC (method described earlier in specification) to understand its short term stability. Each of the formulations was found to be stable, with no increase in impurities after 2 or 24 hours (Table 31).

| **Table 31: stability of spray dried CSP7** | | | | | | | | | | | | | | | | |
|---|---|---|---|---|---|---|---|---|---|---|---|---|---|---|---|---|
| | **100 % LTI-03** | | | **100 % LTI-03** | | | **75 % CSP7** | | | **75 % CSP7** | | | **75 % CSP7** | | | **WS** |
| | | | | | | | **25 % Leucine** | | | **25 % Trehalose** | | | **25 % Sodium Citrate** | | | |
| Base Used | Ammonium hydroxide | | | Sodium hydroxide | | | Ammonium hydroxide | | | Ammonium hydroxide | | | Ammonium hydroxide | | | NA |
| Time Point | 0 hr | 2 hr | 24 hr | Initial | 2 hr | 24 hr | Initial | 2 hr | 24 hr | Initial | 2 hr | 24 hr | 0 hr | 2 hr | 24 hr | 0 hr |
| Purity (% main peak) | 99.7 | 99.6 | 99.5 | 99.6 | 99.6 | 99.3 | 99.6 | 99.7 | 99.6 | 99.6 | 99.6 | 99.6 | 99.6 | 99.6 | 99.6 | 98.9 |

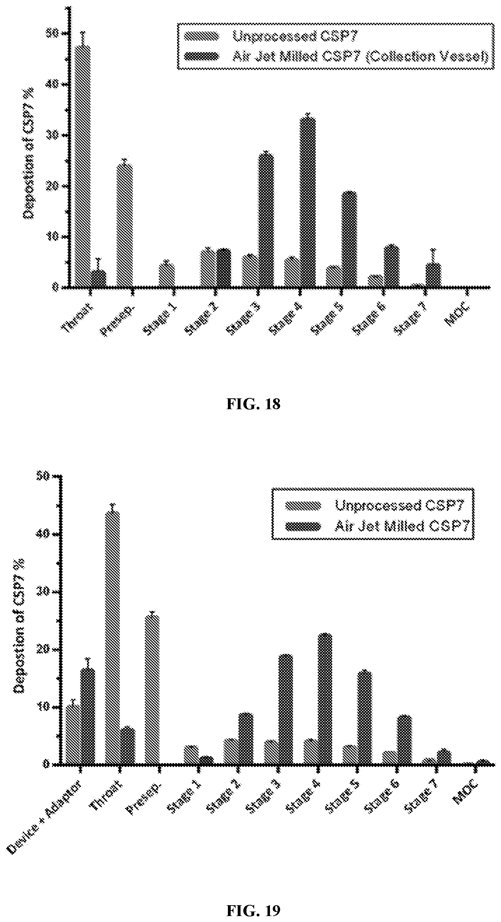

*Aerodynamic particle size distribution of milled CSP7.* To determine aerodynamic particle size distribution of milled CSP7, the amount of powder deposited in various locations of the NGI collector were extracted separately. The delivered dose was measured as the mass of CSP7 that entered the NGI collector after aerosolization, and the amount of CSP7 that was deposited on individual surfaces was extracted and measured separately. The amount of CSP7, either unprocessed or air jet milled (batch 171013) that was left in the capsule, or deposited within the device, adaptor, throat, pre-separator, and stages 1-MOC are shown in FIG. 18 as a percentage of the total amount of CSP7 delivered. The fine particle fraction percentage (FPF%), mass mean aerodynamic diameter (MMAD), and geometric standard deviation (GSD) of both milled and untreated (e.g., unmilled or unprocessed) CSP7 are presented in Table 9.

| **Table 9: Geometric properties of AJM CSP7 powder (Batch 171013)** | | | |
|---|---|---|---|
| | **FPF% (<5 µm)** | **MMAD (µm)** | **GSD (µm)** |
| Unprocessed CSP7 | 19.3 ± 1.4 | 3.5 ± 0.2 | 2.3 ± 0.2 |
| Air Jet Milled CSP7 | 93.7 ± 3.1 | 2.2 ± 0.1 | 1.7 ± 0.0 |

The aerodynamic particle size distribution of the second milled CSP7 batch (171027) was determined as above, except using 4.25 mg of powder per size 3 HPMC capsule. The GSD, FPF%, and MMAD are presented in Table 11, and the percentage of CSP7 deposited in each location is presented in FIG. 19, again as a percentage of the total amount of CSP7 delivered.

| **Table 11: Geometric properties of CSP7 powder batch 171027** | | | |
|---|---|---|---|
| | **FPF% (<5 µm)** | **MMAD (µm)** | **GSD (µm)** |
| Unprocessed CSP7 | 16.4 ± 0.9 | 3.0 ± 0.3 | 2.5 ± 0.0 |
| Air Jet Milled CSP7 | 84.5 ± 0.7 | 2.2 ± 0.0 | 1.9 ± 0.0 |

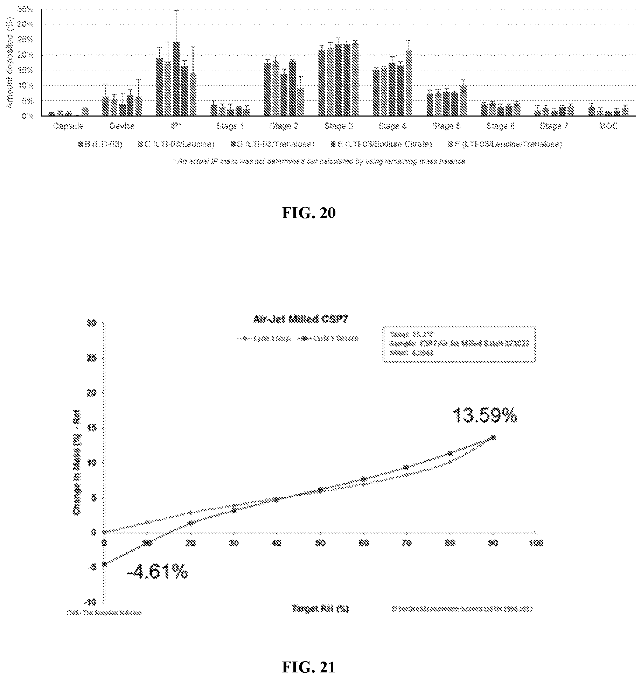

Aerosolization of the spray dried formulations were examined as well (FIG. 20). Each of the formulations exhibited a fine powder fraction of greater than 60%, with each formulation having a MMAD of between 2.5 µm and 3 µm (Table 25). A summary of the results of the analyses of the spray dried formulations, including water content, is presented in Table 25.

| **Table 25: Analytical summary of spray dried CSP7 mixtures** | | | | | | |
|---|---|---|---|---|---|---|
| **Lot ID** | | **BREC1923-001B** | **BREC1923-001C** | **BREC1923-001D** | **BREC1923-001E** | **BREC1923-001F** |
| **Formulation** | | 100% CSP7 | 75/25 CSP7/Leucine | 75/25 CSP7/Trehalose | 75/25 CSP7/Sodium Citrate | 75/15/10 CSP7/ Leucine/Trehalose |
| **Water Content (wt %)** | | 2.1 ±0.1 | 1.8 ±0.0 | 2.1 ±0.0 | 2.5 ±0.1 | 1.8 ±0.1 |
| **Assay (mg A/g, % target)** | | 1059 ±14 (106 %) | 782 ±0 (104%) | 784 ±3 (105 %) | 745 ±9 (99 %) | 757 ±12 (101 %) |
| **Purity (%main peak)** | | 99.8 ±0.3 | 99.9 ±0.2 | 99.6 ±0.1 | 99.8 ±0.1 | 99.8 ±0.0 |
| **Particle Morphology (SEM)** | | Collapsed shells with some fragments | Collapsed shells | Collapsed shells | Collapsed shells with some fragments | Collapsed shells with some fragments |
| **Physical State (PXRD)** | | Amorphous | Primarily Amorphous w/ crystalline leucine character | Amorphous | Amorphous | Primarily Amorphous w/ crystalline leucine character |
| **Thermal Properties(DSC) Midpoint Tg (°C)** | | 132.4 ±6.3 | 97.4 ±1.6 | 77.1 ±2.2 | 80.4 ±0.4 | 79.6 ±2.3 |
| **Aerosol Results** | **MMAD (µm)** | 2.97 ±0.16 | 2.95 ±0.05 | 2.83 ±0.04 | 2.94 ±0.06 | 2.46 ±0.26 |
| | **GSD** | 1.8±0.1 | 1.7±0.0 | 1.8±0.2 | 1.7±0.0 | 1.7±0.0 |
| | **EF (%)** | 93 ±4 | 93 ±2 | 95 ±4 | 93 ±2 | 91 ±6 |
| | **FPF (%)** | 64 +7 | 67 ±6 | 65 ±9 | 69 ±3 | 77 ±9 |
| MMAD - mass median aerodynamic diameter | | | | | | |
| GSD - geometric standard deviation | | | | | | |
| EF - emitted fraction | | | | | | |
| FPF - fine particle fraction | | | | | | |

*Determination of moisture content of milled CSP7.* Under the same conditions used to analyze the bulk CSP7, air jet milled CSP7 powder (batch 171027) was analyzed using Dynamic Vapor Sorption (FIG. 21). Similar to the bulk neat powder, the milled CSP7 had a moisture desorption of 4.61% in relative humidity of 0% (FIG. 21).The KF-V analysis which found a moisture content of 4.9% (Table 7), though there was an increase in mass change to 13.59% when the relative humidity of 90% (FIG. 21).

| **Table 7: KF-C of CSP7 batch 171027** | |
|---|---|
| **Test** | **Moisture Content** |
| 1 | 4.8% |
| 2 | 4.9% |
| 3 | 5.0% |
| **Average** | **4.9%** |

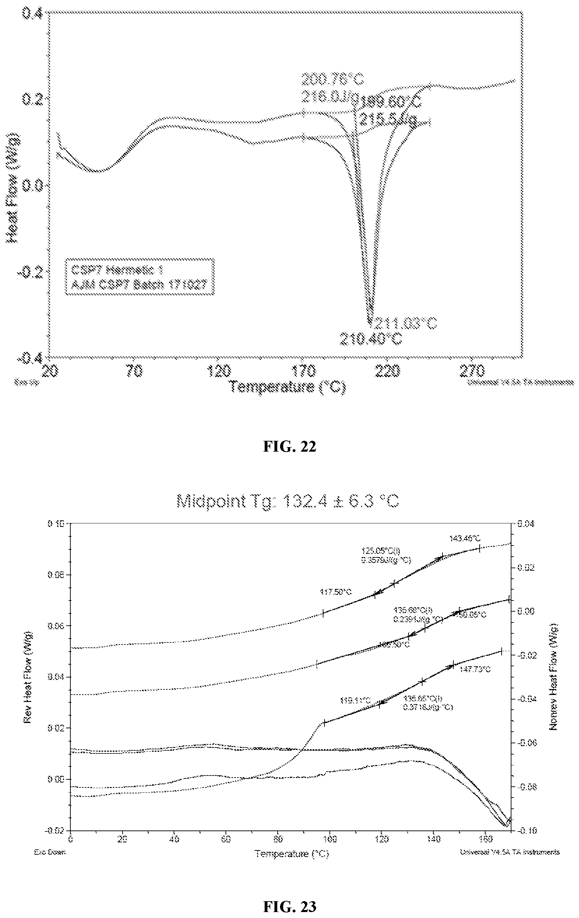

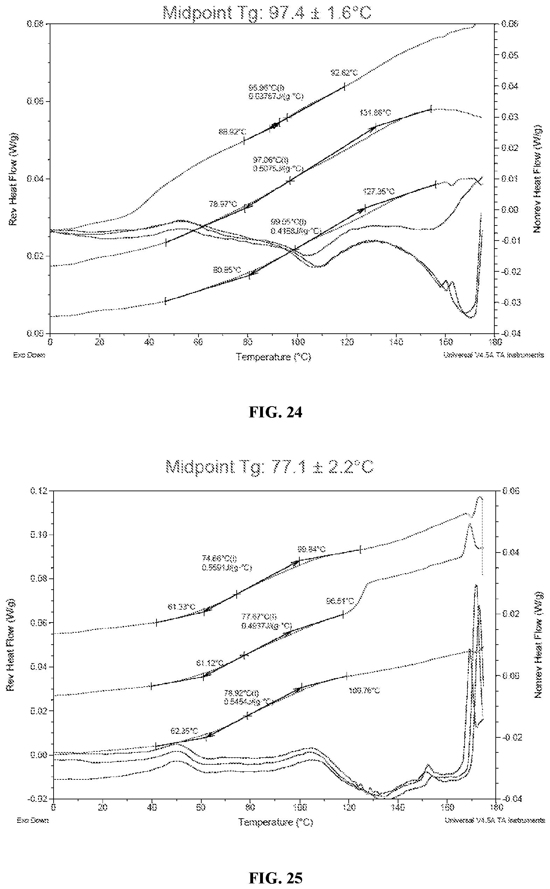

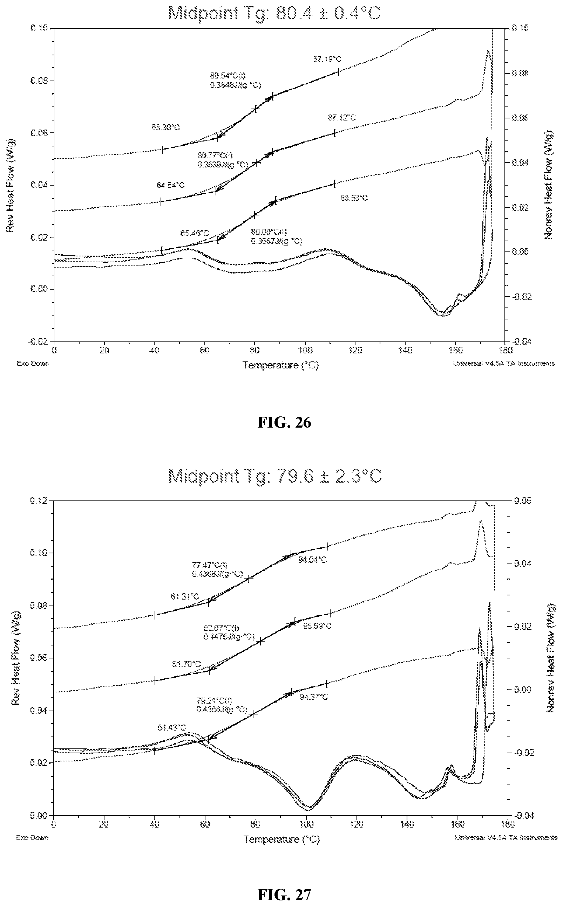

*Thermogravimetric analysis of reduced particle size CSP7 powders.* Thermogravimetric analysis was performed on milled CSP7 (batch 171027) in an identical manner to bulk CSP7, and it was found that the milled CSP7 had very similar properties to the unprocessed CSP7 (FIG. 22). The thermal properties of the spray dried formulations were also evaluated and are shown in Table 24 and FIGS. 23-27 (summarized in FIG. 28). Notably, the midpoint Tg of the mixed formulations was significantly lower than the spray dried CSP7 alone (Compare 001C-F to 001B in Table 24).

| **Table 24: Thermal properties of spray dried CSP7 formulations** | | | |
|---|---|---|---|
| **Lot Number BREC-1923-** | **Formulation (w/w%)** | **Midpoint Tg (°C)** | **Other Recorded Thermal Events** |
| **001B** | 100% LTI-003 | 132.4 ± 6.3 | Exo at 170°C |
| **001C** | 75/25 CSP7/Leucine | 97.4 ± 1.6 | Exo at 165°C followed by Endo at 180°C |
| **001D** | 75/25 CSP7/Trehalose | 77.1 ± 2.2 | Exo at 130°C followed by Endo at 170°C |
| **001E** | 75/25 CSP7/Sodium Citrate | 80.4 ± 0.4 | Exo at 150°C followed by Endo at 170°C |
| **001F** | 75/15/10 CSP7/Leucine/Trehalose | 79.6 ± 2.3 | Exo at 100 and 150°C followed by Endo at 155 and 170°C |

The results presented herein indicate that a variety of methods for reducing the particle size of CSP7 powder are effective, and that the resulting powders exhibit very similar characteristics.

### Example 4 - Treatment of bleomycin induced lung fibrosis by inhalation of CSP7 dry powder

*Induction and CSP7 treatment of fibrosis in mice.* Lung fibrosis was induced in mice by treatment with Bleomycin. Mice were dosed with 0.8 U/kg bleomycin intranasally and wait for 14 days to develop disease before treatment. Mice were then left untreated, or treated by dry powder inhalation of CSP7 for 12 minutes, or treated by dry powder inhalation of CSP7 for 60 minutes. Mice were euthanized at the last day of the treatment and lungs were removed, flash frozen, and stored at -80°C. Flash frozen lungs were weighed prior to further analysis (FIG. 29).

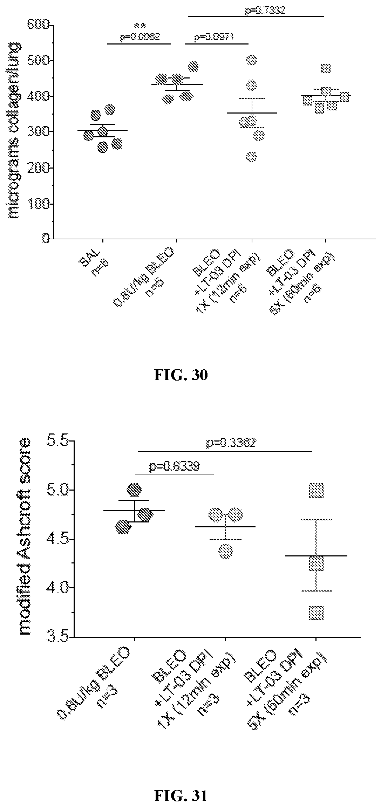

Lung tissues were homogenized and collagen content was analyzed using the Quickzyme collagen assay (FIG. 30). Bleomycin induction of fibrosis resulted in a significant increase in collagen in the lungs compared to saline treatment (P = 0.0062) (FIG. 30). The Ashcroft score, a measure of lung fibrosis in mice, was lower in mice treated with CSP7 following bleomycin induction of lung fibrosis (FIG. 31).

RNA was also prepared from the homogenized lung tissues and used as described above.

### Example 5 - Formulation suspension for intramuscular/subcutaneous injection (for reference, not claimed)

| **Table 25: Composition of CSP7 suspension** | |
|---|---|
| | |
| Sterile Water | - |
| CSP7 (Air-jet-milled*) | 1.2-1.4 mg/ml |
| CMC (low viscosity) | 1.5% (w/v) |
| Poloxamer 188 | 0.2% (w/v) |
| Tris | 20 mM |
| NaCl | 0.7% (w/v) |
| 1 N HCl | ~ 285 µl to pH 7 |

| | |
|---|---|
| *: Air Jet Milled CSP7 exhibited the geometric particle size is Dv(10) = 0.75 µm; Dv(50) = 1.93 µm; Dv(90) = 4.29 µm, measured by laser diffraction. | |

For preparation:
1. Preparing 20 mM Tris buffer (should be pH ~10.3)
2. Dissolve 1.5% (w/w) CMC into 20 mM Tris buffer, with adding 0.2% (w/w) Poloxamer 188. Overnight stir at ~600 rpm
3. Add 0.7% (w/w) NaCl into the CMC solution
4. Adjust the solution pH to 7 by adding ~28.5µl/ml of 1N HCl
5. Weigh and add certain amount of the air jet milled (collected from the collection bag in order to get a small particle size fractions) CSP7 powder in a clean vial
6. Use clean rod to grind the powder first, make sure there is no visibly agglomerated particles
7. Gradually add the prepared solution into the vial with milling/grinding with the rod
8. When the powder is completely wet and no visible agglomerated particles are found, q.s. to the target volume

### Result:

| **Table 26: Characterization of CSP7 suspension** | | | | | | |
|---|---|---|---|---|---|---|
| **Actual Conc. of CSP7** | **pH** | **Osmolarity** | **Chemical Potency at Day 1** | **Chemical Potency at Day8** | | |
| 1.35 mg/ml | 6.99 | 288 mOsm/kg | 101.5% | -20 °C: 99.9% | 4 °C: 101.7% | RT: 85.5% |

### Example 6 - Formulation solution for intramuscular/subcutaneous injection (for reference, not claimed)

For Preparation:
1. Preparing 20 mM Tris buffer (should be pH ~10.3)
2. Dissolve 1.5% (w/w) CMC into 20 mM Tris buffer, with adding 0.2% (w/w) Poloxamer 188. Overnight stir at ~600 rpm
3. Add 0.7% (w/w) NaCl into the CMC solution.
4. Add 1.2-1.4 mg/ml CSP7 (unprocessed powder) to the solution, vortex to dissolve. pH should be around 9, then adjust pH to 8.2-8.5 with adding ~50 µl 1N HCl

| **Table 27: Composition of CSP7 solution** | |
|---|---|
| | |
| Sterile Water | - |
| CSP7 | 1.2-1.4 mg/ml |
| CMC (low viscosity) | 1.5% (w/v) |
| Poloxamer 188 | 0.2% (w/v) |
| Tris (called Trizma) | 20 mM |
| NaCl | 0.7% (w/v) |
| 1N HCl | ~50 µl to pH 8.2-8.5 |

### Result:

| **Table 28: Characterization of CSP7 solution** | | | | | | |
|---|---|---|---|---|---|---|
| **Actual Conc. of CSP7** | **pH** | **Osmolarity** | **Chemical Potency at Day 0** | **Chemical Potency at Day 7** | | |
| 1.18 mg/ml | 8.36 | 286 mOsm/kg | 101.5% | -20 °C: 99.6% | 4 °C: 95.6% | RT: 20.8% |

### Example 7 - Pre-formulation studies for polypeptide variants

Solubility study of variants was performed by adding variants powders into solvent in concentration of 5 mg/ml and then vortexing for 3 min. Observe the appearance of solutions in 5 min. Add more powder (~5mg/ml each time) and repeat vortex and observe until precipitation or gelation occurs.

### Results:

| **Table 29.** pH-solubility of variants | | | | | | | |
|---|---|---|---|---|---|---|---|
| pH | Concentration (mg/ml) | | | | | | |
| | SEQ ID NO.2 | SEQ ID NO.4 | SEQ ID NO.7 | SEQ ID NO.8 | SEQ ID NO.10 | SEQ ID NO.21 | SEQ ID NO.22 |
| 4.5 | 0.15 ± 0.003^{a} | 25.4 ± 0.8^{a} | 32.0 ± 3.3^{b} | 24.2 ± 3.7^{b} | 33.9 ± 0.9^{a} | 0.62 ± 0.04^{a} | 57.17 ± 9.1^{a} |
| 5.5 | 0.16 ± 0.015^{a} | 4.0 ± 0.2^{a} | 22.3 ± 0.1^{a} | 23.6 ± 0.6^{a} | 3.6 ± 0.8^{a} | 0.14 ± 0.02^{a} | 2.98 ± 0.1^{a} |
| 6.5 | 0.16 ± 0.012^{a} | 5.9 ± 0.4^{a} | 30.5 ± 2.0^{b} | 27.3 ± 1.2^{a} | 5.5 ± 0.7^{a} | 0.26 ± 0.07^{a} | 3.49 ± 0.6^{a} |
| 7.5 | 0.31 ± 0.013^{a} | 16.7 ± 0.5^{a} | 35.3 ± 0.6^{b} | 28.3 ± 2.2^{a} | 19.1 ± 0.1^{a} | 0.25 ± 0.03^{a} | 61.51 ± 1.7^{a} |
| 8.5 | 1.43 ± 0.019^{a} | 10.8 ± 1.1^{a} | 23.8 ± 0.4^{b} | 20.6 ± 2.4^{a} | 14.6 ± 1.6^{a} | 0.17 ± 0.04^{a} | 29.13 ± 3.5^{a} |

| | | | | | | | |
|---|---|---|---|---|---|---|---|
| ^{a} Precipitate: centrifuge solutions, then aliquot supernatant and dilute with buffer. ^{b} High viscous solution: take aliquot of solution and dilute with buffer. | | | | | | | |

| **Table 30:** Appearance of solutions at pH 4.5 | | | | | | | | | | | | |
|---|---|---|---|---|---|---|---|---|---|---|---|---|
| SEQ ID NO | Concentration (mg/ml) | | | | | | | | | | | |
| | 5 | 10 | 15 | 20 | 25 | 30 | 35 | 40 | 45 | 50 | 55 | 60 |
| 4 | Clear | Clear | Clear | Clear | Clear | Translucent and colorless precipitate, viscous | - | - | - | - | - | - |
| 7 | Clear | Clear | Clear | Clear | Clear | Clear, slightly viscous | Clear Viscous | Gel | - | - | - | - |
| 8 | Clear | Clear | Clear | Clear | Gel | - | - | - | - | - | - | - |
| 10 | Clear | Clear | Clear | Clear | Clear | Translucent, colorless precipitate | Translucent and colorless precipitate, viscous | - | - | - | - | - |
| 21 | Turbid, viscous | - | - | - | - | - | - | - | - | - | - | - |
| 22 | Clear | Clear | Clear | Clear | Clear | Clear | Clear | Clear | Clear | Clea r | Clear | Cle ar |

| **Table 31:** Appearance of solutions at pH 5.5 | | | | | |
|---|---|---|---|---|---|
| SEQ ID NO | Concentration (mg/ml) | | | | |
| | 5 | 10 | 15 | 20 | 25 |
| 4 | Turbid, viscous | - | - | - | - |
| 7 | Clear | Clear | Clear | Clear, viscous | Turbid, viscous |
| 8 | Clear | Clear, slightly viscous | Clear, slightly viscous | Translucent and colorless precipitate, viscous | Turbid, viscous |
| 10 | Turbid, viscous | - | - | - | - |
| 21 | Turbid, viscous | - | - | - | - |
| 22 | Turbid, viscous | - | - | - | - |

| **Table 32:** Appearance of solutions at pH 7.5 | | | | | | | | | | | | | |
|---|---|---|---|---|---|---|---|---|---|---|---|---|---|
| SEQ ID NO | Concentration (mg/ml) | | | | | | | | | | | | |
| | 5 | 10 | 15 | 20 | 25 | 30 | 35 | 40 | 45 | 50 | 55 | 60 | 65 |
| 4 | Clear | Clear | Translucent and colorless precipitate, viscous | Translucent and colorless precipitate, viscous | - | - | - | - | - | - | - | - | - |
| 7 | Clear | Clear | Clear | Clear | Clear | Clear, slightly viscous | Clear, viscous | Clear, viscous | Translucent and colorless precipitate, viscous | - | - | - | - |
| 8 | Clear | Clear | Clear | Clear, slightly viscous | Clear, slightly viscous | Clear, viscous | Translucent and colorless precipitate, viscous | - | - | - | - | - | - |
| 10 | Clear | Clear | Clear | Clear | Transluc ent, colorless precipitat e, viscous | - | - | - | - | - | - | - | - |
| 21 | Turbid, viscous | - | - | - | - | - | - | - | - | - | - | - | - |
| 22 | Clear | Clear | Clear | Clear | Clear | Clear | Clear | Clear | Clear | Cle ar | Cle ar | Cle ar | Tur bid |

| **Table 33**: Appearance of solutions at pH 8.5 | | | | | | | | |
|---|---|---|---|---|---|---|---|---|
| SEQ ID NO | Concentration (mg/ml) | | | | | | | |
| | 5 | 10 | 15 | 20 | 25 | 30 | 35 | 40 |
| 4 | Clear | Clear | Translucent and colorless precipitate, viscous | Translucent and colorless precipitate, viscous | - | - | - | - |
| 7 | Clear | Clear | Clear | Clear | Clear | Clear, viscous | Clear, viscou s | Turbid, viscous |
| 8 | Clear | Clear | Clear | Clear | Translucent and colorless precipitate, viscous | Translucent and colorless precipitate, viscous | - | - |
| 10 | Clear | Clear | Clear | Turbid, viscous | - | - | - | - |
| 21 | Turbid viscous | - | - | - | - | - | - | - |
| 22 | Clear | Clear | Clear | Clear | Clear, viscous | Turbid, viscous | - | - |

### Example 8 - Pre-formulation study of CSP7 (ammonium counterion) form

Solubility of neat (i.e., not milled) CSP7 ammonium counterion form (Table 35) was performed by adding excess amount of peptide powder into 3 mL different pH buffers (Table 36) and mixing on orbital shaker at 100 rpm for 24 hours at room temperature.

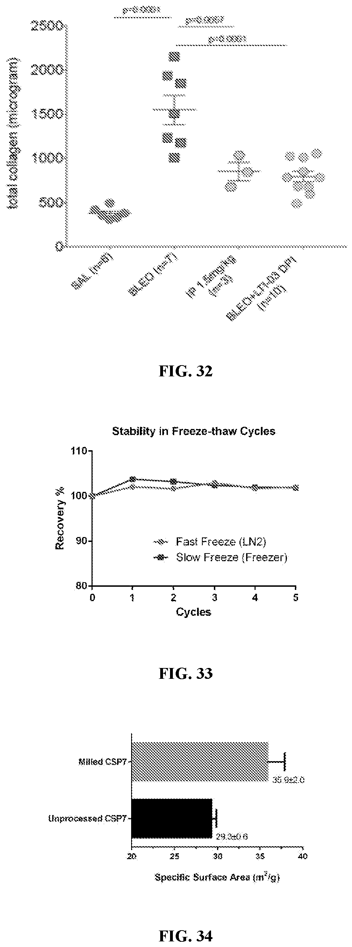

For the freeze-thaw stability study (FIG. 33), 0.1 mg/mL CSP7 (ammonium counterion) peptide in phosphate buffer system (PBS, pH 7.4) was aliquoted into 15 mL/vial for fast and slow freezing, respectively. For fast and slow freezing, samples were either immersed into liquid nitrogen for at least 5 min or put into -20 °C freezer for at least 1.5 hours to ensure that the aliquot in the vial was frozen completely before then being thawed to room temperature. Each sample underwent 5 freeze-thaw cycles. Recovery % represents the percentage of each sample concentration compared to the original (untreated) concentration.

The solubility and freeze-thaw samples were filtered through a 0.45 µm membrane prior to being assayed by performing High Performance Liquid Chromatography (HPLC, Thermo Fisher Scientific, Fair Lawn, NJ). Briefly, samples were analyzed using Dionex 3000 HPLC system equipped with a Waters^{®} reversed phase C18 column 2.5 µm, 150 mm × 4.60 mm. The HPLC column was heated to 60 °C for testing, and the peptide was detected at a wavelength of 215 nm and a 1 mL/min flow rate. The two mobile phases were A (0.1% acetic acid in water) and B (0.1% acetic acid in acetonitrile). The injection volume was 20 µL and the standard curve was plotted from 0.01 -1 mg/ml.

**Table 34. HPLC method details.**

| | | | |
|---|---|---|---|
| Separation Column | XSelect CSH C18, 4.6 x 150mm 2.5um | | |
| Mobil Phase A | 0.1% Acetic acid in H2O | | |
| Mobil Phase B | 0.1% Acetic acid in ACN | | |
| Injection Volume | 20 µL | | |
| Run Time | 48 min | | |
| Column Temp. | 60 °C | | |
| Flow | 1.0 mL/min | | |
| Wavelength | 215 nm | | |
| Gradient | Time (min) | A% | B% |
| | 0 | 90 | 10 |
| | 30 | 75 | 25 |
| | 30.1 | 20 | 80 |
| | 39 | 20 | 80 |
| | 39.1 | 90 | 10 |
| | 48 | 90 | 10 |

**Table 35. pH-solubility profile of CSP7 (ammonium counterion).**

| **pH** | **Conc. (mg/mL)** |
|---|---|
| 3 | 0.17 |
| 4 | 0.07 |
| 5 | 0.05 |
| 6 | 0.07 |
| 7 | 0.10 |
| 8 | 0.38 |
| 9 | 2.30 |
| 10 | 7.87 |
| 11 | 14.43 |

**Table 36. Buffer system and amount of CSP7 peptide added in 3 mL buffers at each pH**

| **pH** | **Buffer System** | **Add ~X mg to 3 ml** |
|---|---|---|
| 3 | 100 mM Citrate | 2 mg |
| 4 | 100 mM Acetate | 2 mg |
| 5 | 100 mM Acetate | 1 mg |
| 6 | 20 mM Citrate | 1 mg |
| 7 | 30 mM Phosphate | 2 mg |
| 8 | 100 mM Tris | 4 mg |
| 9 | 100 mM Tris | 12 mg |
| 10 | 25 mM Carbonate | 30 mg |
| 11 | 25 mM Carbonate | 40 mg |

### Example 9. Characterization and stability study of milled CSP7 (ammonium counterion) powder as Lot# UTA181028

A Model 00 Jet-O-Mizer^{™} (also known as Aljet mill, Fluid Energy, Telford, PA) was used to mill the CSP7 peptide. The feed rate, pushing pressure, and grinding pressure are 1 g/min, 60 psi, and 70 psi, respectively (Table 37). The batch size is 20 g, and the milled powder is collected from different sections of the jet mill, including: the tube after grinding chamber (bfC), the cyclone (C), the collection vessel adapter (D), the collection bag adapter (E), the collection bag (H), and the collection vessel (G). The collected powder was blended for 10 min using a Turbula mixer (Glen Mills Inc., Clifton, NJ, USA).

**Table 37. Parameters for milling CSP7 (ammonium counterion) powder.**

| **Parameter** | **Value** |
|---|---|
| Feeding Rate | 1 g/min |
| Pushing Pressure | 60 psi |
| Grinding Pressure | 70 psi |

**Specific surface area of milled and CSP7 ammonium counterion neat powder.** Specific surface areas of milled and unprocessed CSP7 powder are analyzed using Monosorb Rapid Surface Area Analyzer Model MS-21 (Quantachrome Instruments, Boynton Beach, FL) with single point BET method (FIG. 34). Samples are outgassed at 25 °C with nitrogen gas at 20 psi for 20-24 hours to remove water and other impurity molecules on the surface. A mixture of nitrogen/helium (50:50 v/v) is used as the adsorbate, and the equipment is calibrated with nitrogen before the test.

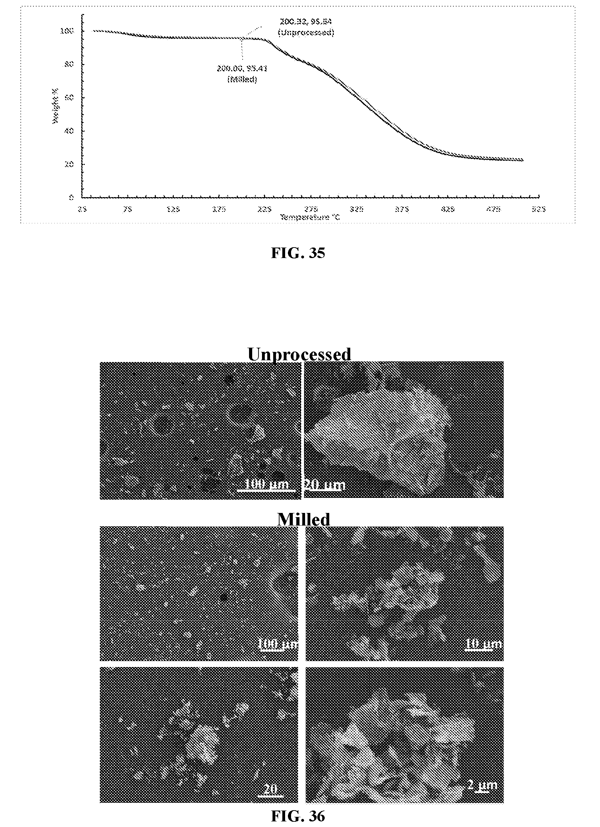

**Thermogravimetric Analysis of milled and CSP7 ammonium counterion neat powder.** Using the method described in paragraph [0021], except that the starting temperature was 35 °C instead of 25 °C. The results are shown in FIG. 35.

**Scanning Electron Microscopy (SEM) Images of milled and neat CSP7 (ammonium counterion) powder.** The morphology of CSP7 (FIG. 36) is analyzed with Zeiss Supra 40VP SEM (Carl Zeiss Microscopy GmbH, Jena, Germany). Samples are mounted on aluminum SEM stubs by a carbon conductive tape and coated with 12 nm of platinum/palladium (Pt/Pd) using a Cressington sputter coater 208 HR (Cressington Scientific Instruments Ltd., Watford, UK). Images are taken for neat (i.e., unprocessed) and post-milled CSP7 samples.

**Stability study for milled CSP7 (ammonium counterion) from Lot# UTA181028.** The stability of milled CSP7 powder was investigated under different storage conditions for a period of up to 6 months. Milled CSP7 peptide is packaged as two forms, bulk milled powder and encapsulated milled powder. For storage as bulk milled powder, 0.21-0.24 g peptide is filled in 20 mL scintillation vials (Kimble^{®}, DWK Life Sciences, Millville, NJ, US) and stored within a heat-sealed foil pouch (Impak Corp, Los Angeles, CA, US) containing two bags of 1 g silica gel desiccant (Tyvek^{®}, Sorbco Packaging LLC, Belen, NM, US) in each pouch. Milled peptide powder is also encapsulated in size 3 HPMC capsules (Capsugel, Morristown, NJ, US) in a weight of 11 ± 5% mg, and 22-26 capsules are then packaged in HDPE bottles (Drug Plastic, Boyertown, PA, US), followed by sealing the HDPE bottle within a foil pouch (without desiccant). Packages are stored in stability chambers at the following storage conditions: -20 °C, 25 °C/60% RH, and 40 °C/75% RH. Samples are removed for testing at 1, 3, and 6 months (Table 38). For tests except the aerodynamic particle size distribution, encapsulated powder was removed from the capsule and mixed in a glass vial by rotating the vial.

**Table 38. Tests schedule for stability study.**

| **Condition** | **Time (Months)** | | | |
|---|---|---|---|---|
| | **Initial** | **1** | **3** | **6** |
| -20 °C | 1-6 | 1-5 | Not Tested | 1-5 |
| 25 °C/60% RH | | | 1-5 | |
| 40 °C/75% RH | | | 1-7 | 1-6 |

| **Test#** | **Test** | **Tentative Specification** | | **Method** |
|---|---|---|---|---|
| **1** | Appearance | Color and appearance (agglomerate) | | Visual |
| **2** | Assay | % of mass balance | | HPLC |
| **3** | Water Content | % water in sample | | KF-C |
| **4** | GPSD | Dv 10, 50, 90; 1-5 µm% | | LD |
| **5** | APSD | MMAD, GSD, EF%, FPD, FPF% | | NGI |
| **6** | Crystallinity | Graph and % crystallinity | | XRPD |

| | | | | |
|---|---|---|---|---|
| * Acronyms: HPLC: High Performance Liquid Chromatography; KF-C: Karl Fischer Coulometric; GPSD: Geometric Particle Size Distribution; LD: Laser Diffraction; APSD: Aerodynamic Particle Size Distribution; NGI: Next Generation Impactor; SEM: Scanning Electron Microscope; XRPD: X-ray Powder Diffraction. | | | | |

**Appearance of the milled powder.** The appearance of the milled powder was recorded by taking pictures with regular camera (FIG. 30).

**Chemical stability of the milled peptide.** The powders were assayed using HPLC method described in Example 8. Results are shown below in Table 39. Percentage represents the assay amount compared to the mass balance. Assay was adjusted for water content.

**Table 39. Assay of milled CSP7 ammonium counterion by HPLC in stability study.**

| Time Point | -20 °C | | 25 °C/ 60RH | | 40 °C/ 75RH | |
|---|---|---|---|---|---|---|
| Initial | 98.85% | | | | | |

| | Bulk | Encapsulated | Bulk | Encapsulated | Bulk | Encapsulated |
|---|---|---|---|---|---|---|
| 1 Month | 98.9 ± 1.2 | 99.8 ± 1.2 | 98. 8± 1.3 | 100.5 ± 1.4 | 100.1 ± 1.3 | 100.9 ± 1.3 |
| 3 Month | - | | 101.3 ± 0.4 | 100.0 ± 0.2 | 102.1 ± 0.2 | 96.9 ± 0.4 |
| 6 Month | 100.9 ± 0.3 | 101.5 ± 0.2 | 100.9 ± 0.2 | 101.7 ± 0.3 | 101.9 ± 0.1 | 100.2 ± 0.6 |

**Moisture content of CSP powder.** Coulometric Karl Fischer (Mettler Toledo C20 Leicester, OH, US) is used to determine the moisture content in the peptide powders (Table 40). The reliability of the equipment is tested with a Karl Fischer water content standard (Hydranal TM water standard, Honeywell, Charlotte, NC, US). A known amount of powder is suspended in anhydrate methanol (Sigma, St. Louis, MO) and the suspension is injected to the anolyte solution (Hydranal^{™}-Coulomat AG, H Honeywell, Charlotte, NC, US) to trigger the titration in the presence of the catholyte solution (Hydranal^{™}-Coulomat CG, H Honeywell, Charlotte, NC, US). The result is recorded as the difference of water content in sample subtracting the blank anhydrous methanol solution.

**Table 40. Moisture content in stability study powder samples.**

| Time Point | -20 °C | | 25 °C/ 60RH | | 40 °C/ 75RH | |
|---|---|---|---|---|---|---|
| Initial | 4.18 ± 0.05% | | | | | |

| | Bulk | Encap. | Bulk | Encap. | Bulk | Encap. |
|---|---|---|---|---|---|---|
| 1 Month | 4.31 ± 0.29% | 4.94 ± 0.15% | 3.66 ± 0.33% | 4.43% | 3.91 ± 0.03% | 4.73 ± 0.08% |
| 3 Month | - | | 4.56 ± 0.31% | 5.51 ± 0.37% | 4.54 ± 0.08% | 5.86 ± 0.14% |
| 6 Month | 4.68 ± 0.08 | 5.61 ± 0.27 | 4.42 ± 0.32 | 5.76 ± 0.06 | 4.71 ± 0.21 | 6.20 |
| | | | | | | 0.18 |

**Geometric particle size distribution.** GPSD of CSP7 powder was analyzed before and after milling using a Sympatec HELOS laser diffraction instrument (Sympatec GmbH, Germany) equipped with RODOS dispersion. Measurements are taken every 10 ms following powder dispersion at 3 bar. Measurements that are between 5 and 25% optical density were averaged to determine particle size distribution. The particle sizes by volume are reported at percentiles of 10, 50, and 90 (e.g., Dv 10, Dv 50, and Dv 90), respectively, as well as the percentages of particles falling into 1-5 µm size range. The results are shown in 41.

**Table 41. Geometric particle size distribution of milled CSP7 (ammonium counterion) in the stability study**

| Lot#UTA181028 | | | Dv 10 (µm) | Dv 50 (µm) | Dv 90 (µm) | % 1-5 µm |
|---|---|---|---|---|---|---|
| Unprocessed | | | 1.2 ± 0 | 4.5 ± 0.1 | 15.7 ± 0.8 | 47.0 ± 0.9 |
| Initial | | | 0.8 ± 0 | 2.2 ± 0 | 4.6 ± 0 | 77.9 ± 0.3 |
| 1 Month | Bulk | -20 °C | 0.8 ± 0 | 2.2 ± 0 | 4.9 ± 0.1 | 74.9 ± 0.7 |
| | | 25 °C/ 60RH | 0.8 ± 0 | 2.1 ± 0 | 4.8 ± 0 | 74.3 ± 0.3 |
| | | 40 °C/ 75RH | 0.8 ± 0 | 2.1 ± 0 | 4.8 ± 0 | 74.6 ± 0 |
| | Encapsulated | -20 °C | 0.8 ± 0 | 2.1 ± 0 | 4.8 ± 0.1 | 74.1 ± 0.1 |
| | | 25 °C/ 60RH | 0.8 ± 0 | 2.1 ± 0.1 | 4.7 ± 0.1 | 73.6 ± 0.8 |
| | | 40 °C/ 75RH | 0.8 ± 0 | 2 ± 0 | 4.7 ± 0.1 | 72.8 ± 0.4 |
| 3 Month | Bulk | 25 °C/ 60RH | 0.8 ± 0 | 2.1 ± 0.1 | 4.7 ± 0.1 | 73.7 ± 0.3 |
| | | 40 °C/ 75RH | 0.8 ± 0 | 2.2 ± 0 | 4.8 ± 0 | 75 ± 0.5 |
| | Encapsulated | 25 °C/ 60RH | 0.8 ± 0 | 2.1 ± 0 | 4.7 ± 0 | 73.6 ± 0.4 |
| | | 40 °C/ 75RH | 0.8 ± 0 | 2.1 ± 0 | 4.7 ± 0 | 73.9 ± 0.1 |
| 6 Month | Bulk | -20 °C | 0.8±0 | 2.2±0.1 | 4.9±0.1 | 73.8±0.5 |
| | | 25 °C/ 60RH | 0.8±0 | 2.2±0 | 4.9±0 | 74±0.4 |
| | | 40 °C/ 75RH | 0.8±0 | 2.1±0 | 4.8±0.1 | 73.6±0.6 |
| | Encapsulated | -20 °C | 0.8±0 | 2.2±0 | 4.9±0 | 74.4±0.1 |
| | | 25 °C/ 60RH | 0.8±0 | 2.1±0 | 4.8±0 | 73.9±0.3 |
| | | 40 °C/ 75RH | 0.8±0 | 2.1±0 | 4.9±0 | 73.8±0.3 |

**Aerodynamic particle size distribution:** The aerodynamic particle size distribution was evaluated by performing NGI as described in paragraph [00215], except that the powder weight in the tested capsule was 11±5% mg and pre-separator was removed in the assembly of the NGI in stability study. The results are given in Table 42.

**Table 42. Aerodynamic particle size distribution of milled CSP7 (ammonium counterion) in the stability study.**

| Lot#UTA181028 | | | FPD (mg) | FPF% | EF% | MMAD (µm) | GSD |
|---|---|---|---|---|---|---|---|
| Unprocessed | | | 4.2 ± 0.3 | 48.4 ± 3.1 | 92.8 ± 1.4 | 3.09 ± 0.07 | 4.67 ± 0.09 |
| Initiate | | | 8.3 ± 0.5 | 93.3 ± 3.3 | 83.5 ± 0.1 | 1.58 ± 0.10 | 1.91 ± 0.03 |
| 1 Month | Bulk | -20 °C | 8.0 ± 0.4 | 88.8 ± 2.9 | 84 ± 1.4 | 1.86 ± 0.16 | 1.86 ± 0.02 |
| | | 25 °C/ 60RH | 7.7 ± 0.5 | 92.2 ± 1.0 | 80.7 ± 1.8 | 1.69 ± 0.09 | 1.86 ± 0.01 |
| | | 40 °C/ 75RH | 8.0 ± 0.3 | 91.8 ± 0.6 | 85.2 ± 1.4 | 1.79 ± 0.08 | 1.86 ± 0.02 |
| | Encapsulation | -20 °C | 7.3 ± 0.1 | 87.8 ± 0.3 | 83.6 ± 1.3 | 1.85 ± 0.03 | 1.91 ± 0.01 |
| | | 25 °C/ 60RH | 7.6 ± 0.3 | 90.0 ± 1.4 | 81.6 ± 4 | 1.74 ± 0.08 | 1.90 ± 0.02 |
| | | 40 °C/ 75RH | 7.9 ± 0.3 | 89.6 ± 1.3 | 84.7 ± 1 | 1.80 ± 0.15 | 1.90 ± 0.02 |
| 3 Month | Bulk | 25 °C/ 60RH | 7.5 ± 0.5 | 92.2 ± 2.1 | 79.8 ± 0.3 | 1.55 ± 0.09 | 1.84 ± 0.06 |
| | | 40 °C/ 75RH | 7.7 ± 0.3 | 90.4 ± 1.6 | 82.9 ± 0.8 | 1.66 ± 0.18 | 1.91 ± 0.05 |
| | Encapsulation | 25 °C/ 60RH | 7.6 ± 0.3 | 89.4 ± 0.8 | 80.5 ± 1.9 | 1.76 ± 0.05 | 1.85 ± 0.01 |
| | | 40 °C/ 75RH | 7.4 ± 0.3 | 87.6 ± 0.6 | 83.4 ± 2.5 | 1.83 ± 0.06 | 1.92 ± 0.03 |
| 6 Month | Bulk | -20 °C | 7.2 ± 0.1 | 87.1 ± 1.5 | 79.1 ± 1.6 | 1.79 ± 0.10 | 1.90 ± 0.01 |
| | | 25 °C/ 60RH | 7.2 ± 0.2 | 87.1 ± 1.8 | 80.4 ± 2.1 | 1.85 ± 0.08 | 1.86 ± 0.01 |
| | | 40 °C/ 75RH | 7.3 ± 0.1 | 86.2 ± 0.8 | 81.9 ± 1.2 | 1.86 ± 0.01 | 1.92 ± 0.06 |
| | Encapsulation | -20 °C | 7.3 ± 0.1 | 85.3 ± 1.8 | 80.9 ± 1.3 | 1.90 ± 0.13 | 1.95 ± 0.06 |
| | | 25 °C/ 60RH | 7.4 ± 0.4 | 87.9 ± 0.5 | 80.0 ± 0.4 | 1.77 ± 0.02 | 1.90 ± 0.02 |
| | | 40 °C/ 75RH | 7.4 ± 0.0 | 85.6 ± 2.7 | 80.7 ± 1.1 | 1.81 ± 0.11 | 1.94 ± 0.03 |

| | | | | | | | |
|---|---|---|---|---|---|---|---|
| Note: IP: Induction Port; FPD: Fine particle dose (particles <5 µm); FPF (%): Fine particle dose over delivered dose | | | | | | | |

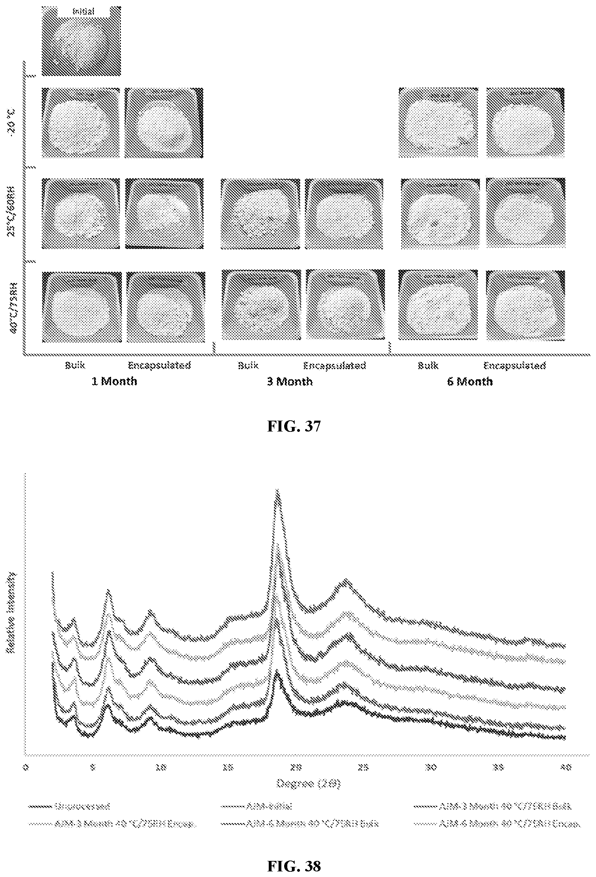

**Crystallinity of the CSP7 powders.** Crystallinity of powders was evaluated by method described in paragraph [0018], and the results are shown in FIG. 38.

### REFERENCES

The following references, to the extent that they provide exemplary procedural or other details supplementary to those set forth herein.
Carvalho et al., "Influence of particle size on regional lung deposition - What evidence is there?" Int. J. Pharma. 406:1-10, 2011.
Hübner, R.-H.; Gitter, W.; El Mokhtari, N. E.; Mathiak, M.; Both, M.; Bolte, H.; Freitag-Wolf, S.; Bewig, B. Standardized quantification of pulmonary fibrosis in histological samples. Biotechniques, 44, 507-11, 514-7, 2008.
Surasarang et al., "Optimization of Formulation for a Novel Inhaled Candidate Therapeutic for Idiopathic Fibrosis," Drug Development and Industrial Pharmacy, 44(2):184-198, 2017.
Tepper, J. S.; Kuehl, P. J.; Cracknell, S.; Nikula, K. J.; Pei, L.; Blanchard, J. D. Symposium Summary: "breathe In, Breathe Out, Its Easy: What You Need to Know about Developing Inhaled Drugs." Int. J. Toxicol. 35, 376-392, 2016.

## Claims

1. A pharmaceutical composition for use in the treatment of an inflammatory disorder or fibrotic condition of the lungs, the pharmaceutical composition comprising a dry powder of a peptide, or a pharmaceutically acceptable salt thereof, wherein the peptide is the amino acid sequence of SEQ ID NO: 2;
wherein the pharmaceutical composition is in the form of a dry powder formulation and to be administered using a dry powder inhaler, and
the pharmaceutical composition is excipient free.

2. The pharmaceutical composition for use of claim 1, wherein the dry powder is produced by a milling process or a spray-drying process, wherein the milling process is air jet milling, ball milling or wet milling.

3. The pharmaceutical composition for use of claim 1, wherein the dry powder comprises less than 10% (by weight) of water.

4. The pharmaceutical composition for use of claim 1, wherein particles in the pharmaceutical composition have a mass median aerodynamic diameter (MMAD) of less than 5 µm.

5. The pharmaceutical composition for use of claim 1, wherein at least 70% of particles in the pharmaceutical composition are less than 5 µm in size.

6. The pharmaceutical composition for use of claim 1, wherein the peptide is at least 95% pure by weight, or at least 99% pure by weight.

7. The pharmaceutical composition for use of any one of claims 1 to 6, wherein the pharmaceutical composition is for use in the treatment of chronic obstructive pulmonary disorder (COPD), acute lung injury, lung infection, chemical-induced lung injury, plastic bronchitis, asthma, acute respiratory distress syndrome (ARDS), inhalational smoke-induced acute lung injury (ISALI), bronchiolitis, bronchiolitis obliterans, a fibrotic condition of the lungs, interstitial lung disease, idiopathic pulmonary fibrosis (IPF) or lung scarring.

8. The pharmaceutical composition for use of any one of claims 1 to 6, wherein the pharmaceutical composition is for use in the treatment of idiopathic pulmonary fibrosis (IPF).

9. The pharmaceutical composition for use of any one of claims 1 to 8, wherein the pharmaceutical composition is to be administered with at least one additional therapeutic.

10. The pharmaceutical composition for use of claim 9, wherein the at least one additional therapeutic is a non-steroidal anti-inflammatory drug (NSAID), a steroid, a disease-modifying antirheumatic drug (DMARD), an immunosuppressive, or a bronchodilator.

11. The pharmaceutical composition for use of any one of claims 1 to 10, wherein the subject is a human.

## Patentansprüche

1. Pharmazeutische Zusammensetzung zur Verwendung bei der Behandlung einer entzündlichen Erkrankung oder eines fibrotischen Zustands der Lunge, wobei die pharmazeutische Zusammensetzung ein Trockenpulver eines Peptids oder eines pharmazeutisch verträglichen Salzes davon umfasst, wobei das Peptid die Aminosäuresequenz der SEQ ID NO:2 ist;
wobei die pharmazeutische Zusammensetzung in Form einer Trockenpulverformulierung ist und unter Verwendung eines Trockenpulverinhalators zu verabreichen ist, und
die pharmazeutische Zusammensetzung frei von Exzipienten ist.

2. Pharmazeutische Zusammensetzung zur Verwendung nach Anspruch 1, wobei das Trockenpulver mit einem Mahlverfahren oder einem Sprühtrocknungsverfahren hergestellt wird, wobei das Mahlverfahren Luftstrahlvermahlung, Kugelvermahlung oder Nassvermahlung ist.

3. Pharmazeutische Zusammensetzung zur Verwendung nach Anspruch 1, wobei das Trockenpulver weniger als 10 Gew.-% Wasser umfasst.

4. Pharmazeutische Zusammensetzung zur Verwendung nach Anspruch 1, wobei Partikel in der pharmazeutischen Zusammensetzung einen medianen massenbezogenen aerodynamischen Durchmesser (MMAD) von weniger als 5 µm aufweisen.

5. Pharmazeutische Zusammensetzung zur Verwendung nach Anspruch 1, wobei mindestens 70% der Partikel in der pharmazeutischen Zusammensetzung eine Größe von weniger als 5 µm aufweisen.

6. Pharmazeutische Zusammensetzung zur Verwendung nach Anspruch 1, wobei das Peptid einen Reinheitsgrad von mindestens 95 Gew.-%, oder einen Reinheitsgrad von mindestens 99 Gew.-% aufweist.

7. Pharmazeutische Zusammensetzung zur Verwendung nach einem der Ansprüche 1 bis 6, wobei die pharmazeutische Zusammensetzung zur Verwendung bei der Behandlung von chronisch obstruktiver Lungenerkrankung (COPD), akuter Lungenverletzung, Lungeninfektion, chemisch-bedingter Lungenschädigung, plastischer Bronchitis, Asthma, akutem Atemnotsyndrom (ARDS), durch Rauchinhalation verursachter akuter Lungenverletzung (ISALI), Bronchiolitis, Bronchiolitis obliterans, einer fibrotischen Erkrankung der Lunge, interstitieller Lungenerkrankung, idiopathischer Lungenfibrose (IPF) oder Lungennarben ist.

8. Pharmazeutische Zusammensetzung zur Verwendung nach einem der Ansprüche 1 bis 6, wobei die pharmazeutische Zusammensetzung zur Verwendung bei der Behandlung von idiopathischer Lungenfibrose (IPF) ist.

9. Pharmazeutische Zusammensetzung zur Verwendung nach einem der Ansprüche 1 bis 8, wobei die pharmazeutische Zusammensetzung mit mindestens einem zusätzlichen Therapeutikum zu verabreichen ist.

10. Pharmazeutische Zusammensetzung zur Verwendung nach Anspruch 9, wobei das mindestens ein zusätzliches Therapeutikum ein nicht-steroidales entzündungshemmendes Arzneimittel (NSAID), ein Steroid, ein krankheitsmodifizierendes Antirheumatikum (DMARD), ein Immunsuppressivum oder ein Bronchodilatator ist.

11. Pharmazeutische Zusammensetzung zur Verwendung nach einem der Ansprüche 1 bis 10, wobei das Individuum ein Mensch ist.

## Revendications

1. Composition pharmaceutique pour utilisation dans le traitement d'un trouble inflammatoire ou d'un état fibrotique des poumons, la composition pharmaceutique comprenant une poudre sèche d'un peptide, ou un sel pharmaceutiquement acceptable de celui-ci, dans laquelle le peptide est la séquence d'acides aminés de SEQ ID NO : 2 ; dans laquelle la composition pharmaceutique est sous la forme d'une formulation de poudre sèche et doit être administrée en utilisant un inhalateur de poudre sèche, et la composition pharmaceutique est sans excipient.

2. Composition pharmaceutique pour utilisation selon la revendication 1, dans laquelle la poudre sèche est produite par un procédé de broyage ou un procédé de séchage par pulvérisation, dans lequel le procédé de broyage est un broyage par jet d'air, un broyage à billes ou un broyage par voie humide.

3. Composition pharmaceutique pour utilisation selon la revendication 1, dans laquelle la poudre sèche comprend moins de 10 % (en poids) d'eau.

4. Composition pharmaceutique pour utilisation selon la revendication 1, dans laquelle les particules de la composition pharmaceutique présentent un diamètre aérodynamique médian en masse (MMAD) inférieur à 5 µm.

5. Composition pharmaceutique pour utilisation selon la revendication 1, dans laquelle au moins 70 % des particules de la composition pharmaceutique présentent une taille inférieure à 5 µm.

6. Composition pharmaceutique pour utilisation selon la revendication 1, dans laquelle le peptide est au moins pur à 95 % en poids ou au moins pur à 99 % en poids.

7. Composition pharmaceutique pour utilisation selon l'une quelconque des revendications 1 à 6, dans laquelle la composition pharmaceutique est destinée à être utilisée dans le traitement du trouble pulmonaire obstructif chronique (BPCO), d'une lésion pulmonaire aiguë, d'une infection pulmonaire, d'une lésion pulmonaire induite par des produits chimiques, d'une bronchite plastique, de l'asthme, du syndrome de détresse respiratoire aiguë (SDRA), d'une lésion pulmonaire aiguë induite par la fumée inhalée (ISALI), d'une bronchiolite, d'une bronchiolite oblitérante, d'un état fibreux des poumons, d'une maladie pulmonaire interstitielle , d'une fibrose pulmonaire idiopathique (FPI) ou d'une cicatrisation pulmonaire.

8. Composition pharmaceutique pour utilisation selon l'une quelconque des revendications 1 à 6, dans laquelle la composition pharmaceutique est destinée à être utilisée dans le traitement de la fibrose pulmonaire idiopathique (FPI).

9. Composition pharmaceutique pour utilisation selon lune quelconque des revendications 1 à 8, dans laquelle la composition pharmaceutique doit être administrée avec au moins un agent thérapeutique supplémentaire.

10. Composition pharmaceutique pour utilisation selon la revendication 9, dans laquelle le au moins un agent thérapeutique supplémentaire est un médicament anti-inflammatoire non stéroïdien (AINS), un stéroïde, un médicament antirhumatismal modificateur de la maladie (DMARD), un immunosuppresseur ou un bronchodilatateur.

11. Composition pharmaceutique pour utilisation selon l'une quelconque des revendications 1 à 10, dans laquelle le sujet est un humain.
